# EUROPEAN PATENT SPECIFICATION

(11) **EP 2 978 409 B1**
(45) Date of publication and mention of the grant of the patent: **10.01.2018**
(21) Application number: 13880074.3
(22) Date of filing: 27.03.2013
(51) Int. Cl.: A61K 9/127, A61P 27/02

(54) **OPHTHALMIC COMPOSITION, METHOD FOR PREPARING THE SAME, AND USE OF THE SAME**
OPHTHALMISCHE ZUSAMMENSETZUNG, VERFAHREN ZUR HERSTELLUNG UND VERWENDUNG DAVON
COMPOSITION OPHTALMIQUE, SON PROCÉDÉ DE PRÉPARATION ET SON UTILISATION

(43) Date of publication of application: 03.02.2016
(73) Proprietor: Comprehensive Drug Enterprises Ltd, Shatin, N.T. Hong Kong (CN); Fudan University, Shanghai 201203 (CN)
(72) Inventor: WEI, Gang, Shanghai 201203 (CN); LEE, Benjamin Tak Kwong, Shatin Hong Kong (CN); ZHANG, Wenjian, Shanghai 201203 (CN); LU, Weiyue, Shanghai 201203 (CN); LAU, Johnson Yiu-Nam, Newport Beach, CA 92660 (US); LAM, Shun Chiu Dennis, Hong Kong (CN)
(74) Representative: Nordmeyer, Philipp Werner
(86) International application number: PCT/CN2013/073259
(87) International publication number: WO 2014/153733

(56) References cited:
- WO-A1-99/34830
- CN-A- 101 028 240
- CN-A- 101 028 240
- CN-A- 102 670 499
- CN-A- 102 670 499
- US-B1- 6 375 960
- US-B1- 6 419 946

## Description

### Field of the Invention

This invention relates to the field of ophthalmology. In particular, the invention relates to ophthalmic compositions, methods for preparing the same, and the related uses.

### Background of the Invention

Dry eye syndrome (DES) is also called keratoconjunctivitis sicca, keratitis sicca, or Xerophthalmia, which is a common ophthalmological disorder of the tear film due to tear deficiency or excessive tear evaporation. It causes damage to the interpalpebral ocular surface associated with symptoms of ocular discomfort. Typical symptoms of dry eye are dryness, burning and persistent irritation. Many people with dry eyes may experience mild irritation with no long-term effects. However, if the condition is not treated or becomes severe, it can cause eye damage, resulting in impaired vision or even loss of vision.

Current approaches for treatment of dry eye include avoidance of irritating factors, tear stimulation and supplementation, increasing tear retention, and eyelid cleansing and anti-inflammation therapy. As a common approach, ocular tear film can be supplemented with artificial tears applied throughout the day. Examples of tear substitute approaches include the use of buffered, isotonic saline solutions and aqueous solutions containing water-soluble polymers that render the solutions more viscous and thus less easily shed by the eye by the washing action of the tear fluid.

However, significant challenges in the treatment of dry eye remain. Some tear substitutes, while temporarily effective, generally require repeated application during waking hours, typically ten to twenty times in one day. Such is not only inconvenient and time consuming, but also not very effective in preventing at least the initiation of dry-eye symptoms. For instance, CN101028240A discloses ophthalmic compositions in the form of an in situ microemulsion / submicro emulsion gel formulation comprising vitamin A, vitamine E, a liquid oil phase, an emulsifier, a gel substrate and a pH regulator. The compositions disclosed in CN101028240A are prepared by mixing said components, adding water, emulsifying, high-pressure homogenizing, filtering and adding a solution containing the gel substrate, adjusting the pH, adding more water and homogenizing.

Although increasing the viscosity of the dry-eye product may extend the product's duration in the eye, the duration can only be extended to a limited extent. Further, viscous ophthalmic drops are sometimes undesirable because they may stick to the eye. See, for example, CN101057966A. Some tear substitutes are not transparent, therefore cannot be applied during daytime.

In view of the above, it is desirable to provide an ophthalmic composition that could reduce the symptoms of dry eye better and that is safe, convenient and economical to use, and/or could be used during daytime without blocking of vision.

### Objects of the Invention

Therefore, it is an object of this invention to resolve at least one or more of the problems as set forth in the prior art. Particularly, it is an object of the current invention to provide an ophthalmic composition for treating at least one of ophthalmologic diseases, such as dry eye syndrome, ocular inflammation, cystoid macular edema, or Glaucoma. It is also an object of the current invention to provide method for preparing the ophthalmic composition. It is also an object of the current invention to provide use of the ophthalmic composition.

### Summary of the Invention

Accordingly, this invention provides an ophthalmic composition including a mixture of lipid nano-dispersion and gel substrate, wherein the lipid nano-dispersion includes a first lipid, a second lipid, and emulsifier; in which the first lipid exists in form of solid lipid as staring material, and the second lipid exists in form of liquid lipid as staring material.

Preferably, the solid lipid includes yellow Vaseline and wool fat.

More preferably, the yellow Vaseline and the wool fat are at weight ratio of (4-10) : 1; further preferably, the yellow Vaseline and the wool fat are at weight ratio of 8 : 1.

Preferably, based on the total volume of the ophthalmic composition, the ophthalmic composition includes 0.1% ~ 6% (w/v) of the first lipid; more preferably, the ophthalmic composition includes 0.1% ~ 0.5% (w/v) of the first lipid.

Preferably, the liquid lipid includes at least one of medium-chain triglycerides, propylene glycol dicaprylate/dicaprate, naphthenic mineral oil, mineral oil, castor oil, polyoxyethylene castor oil, and polyoxyethylene hydrogenated castor oil; more preferably, the liquid lipid includes medium-chain triglycerides; further preferably, the medium-chain triglycerides include caprylic/capric triglyceride.

Preferably, based on the total volume of the ophthalmic composition, the ophthalmic composition includes 0.1% ~ 10% (w/v) of the second lipid; more preferably, the ophthalmic composition includes 0.25% ~ 8% (w/v) of the second lipid.

Preferably, the emulsifier includes at least one oil-phase emulsifier, and optionally, at least one aqueous-phase emulsifier.

Preferably, based on the total volume of the ophthalmic composition, the ophthalmic composition includes 0.1% ~ 6% (w/v) of the at least one oil-phase emulsifier; more preferably, the ophthalmic composition includes 0.2% ~ 3.5% (w/v) of the at least one oil-phase emulsifier.

Preferably, the at least one oil-phase emulsifier includes at least one of Egg yolk phosphatidylcholine, Soybean phospholipids, TegoCare, Hydrogenated soybean phosphatidylcholine, Myverol, and Span; more preferably, the at least one oil-phase emulsifier includes Egg yolk phosphatidylcholine.

Preferably, based on the total volume of the ophthalmic composition, the ophthalmic composition includes 0.1% ~ 8% (w/v) of the at least one aqueous-phase emulsifier; more preferably, the ophthalmic composition includes 0.25% ~ 6% (w/v) of the at least one aqueous-phase emulsifier.

Preferably, the at least one aqueous-phase emulsifier includes at least one of Poloxamer 188, Tween, sodium cholate, Sodium deoxycholate, Cremphor EL, and Solutol® HS; more preferably, the at least one aqueous-phase emulsifier includes Poloxamer 188.

Preferably, the gel substrate includes a gel-forming agent dispersed in a buffer solution; more preferably, the gel substrate further includes a water-retaining agent.

Preferably, the gel-forming agent includes at least one of polyvinyl pyrrolidone, polyacrylates, and polysaccharides; more preferably, the gel-forming agent includes polyvinyl pyrrolidone.

Preferably, based on the total volume of the ophthalmic composition, the ophthalmic composition includes 0.2% ~ 3% (w/v) of the gel-forming agent; more preferably, the ophthalmic composition includes 1% ~ 1.8% (w/v) of the gel-forming agent.

Preferably, the water-retaining agent includes at least one of polyethylene glycol, glycerin, hyaluronic acid, chitosan, chondroitin sulfate, and sodium Hyaluronate; more preferably, the water-retaining agent includes glycerin.

Preferably, based on the total volume of the ophthalmic composition, the ophthalmic composition includes 0.1 % ~ 5% (w/v) of the water-retaining agent; more preferably, the ophthalmic composition includes 0.1% ~ 3% (w/v) of the water-retaining agent.

Preferably, the buffer solution includes at least one of borate saline buffer solution, phosphate buffer solution, Gifford's buffer solution, sodium acetate-boric acid buffer solution, Atking and parting buffer solution, and Feldman buffer solution; more preferably, the buffer solution includes borate saline buffer solution.

Preferably, the ophthalmic composition has a pH in a range of 4~9; more preferably, the ophthalmic composition has a pH in a range of 6~9; further preferably, the ophthalmic composition has a pH in a range of 6~8.

Preferably, the ophthalmic composition has a lipid particle size in a range of 20 ~ 500 nm; more preferably, the ophthalmic composition has a lipid particle size in a range of 50 ~ 400 nm; further preferably, the ophthalmic composition has a lipid particle size in a range of 50 ~ 200 nm.

Preferably, the ophthalmic composition has a Polydispersity Index less than 0.5; more preferably, the ophthalmic composition has a Polydispersity Index less than 0.4; further preferably, the ophthalmic composition has a Polydispersity Index less than 0.3.

Preferably, the ophthalmic composition has a zeta potential in a range of ± 5~50 mV; more preferably, the ophthalmic composition has a zeta potential in a range of ± 10~40 mV; further preferably, the ophthalmic composition has a zeta potential in a range of ± 20~30 mV.

Preferably, the ophthalmic composition includes at least one drug; more preferably, the at least one drug includes at least one of Cyclosporine, Tacrolimus, Flurbiprofen, Triamcinolone Acetonide, Tobramycin, Difluprednate, and Latanoprost.

It is another aspect of this invention to provide a method for preparing the ophthalmic composition, wherein the method includes:
I) independently preparing a lipid nano-dispersion and a gel substrate,
   wherein the preparation of the lipid nano-dispersion includes:
   i) mixing an oil phase dispersion containing a solid lipid, a liquid lipid, an oil-phase emulsifier dispersed in an organic solvent, preferably at a temperature in a range of 60 ~ 90°C, with an aqueous phase solution containing a aqueous-phase emulsifier dissolved in water, preferably at a temperature in a range of 60 ~ 90°C, to obtain a preemulsion, preferably for 3-10min;
   ii) homogenizing the preemulsion, preferably under a pressure in a range of 500 ~ 1500 bar for 6-20 cycles;
   iii) removing the organic solvent from the homogenized preemulsion by evaporation; and
   iv) solidifying the preemulsion, preferably at a temperature in a range of 15 ~ 30°C for 10-60 min and ice bathed for 5-30 min, to obtain the lipid nano-dispersion; and
      wherein the preparation of the gel substrate includes: dispersing a gel-forming agent and a water-retaining agent in a buffer solution; and
II) mixing the lipid nano-dispersion and gel substrate.

It is yet another aspect of this invention to provide the ophthalmic composition for use in preventing or treating dry eye syndrome, ocular inflammation, cystoid macular edema, or Glaucoma.

### Brief description of the drawings

Preferred embodiments of the present invention will now be explained by way of example and with reference to the accompanying drawings in which:
Figure 1 shows the results of single factor experiment, in which Fig. 1A shows the results of particle sizes measured for the lipid nano-dispersions of this invention, and Fig. 1B shows the results of PDI measured;
Figure 2 shows the results of short term stability study of the lipid nano-dispersions A-M, in which Fig. 2A shows the results of particle sizes measured for the lipid nano-dispersions A-M, and Fig. 2B shows the results of PDI measured;
Figure 3 shows the results of long term stability of the lipid nano-dispersion K, in which Fig. 3A shows the results of particle sizes measured for the lipid nano-dispersion K, and Fig. 3B shows the results of PDI measured;
Figure 4 shows a photo of the appearances of the lipid nano-dispersions N (left) and O (right);
Figure 5 shows the results of the optimization of the preparation method of this invention, in which Fig. 5A shows the results of particle sizes measured for the lipid nano-dispersions of this invention, Fig. 5B shows the results of PDI measured, and Fig. 5C shows the results of zeta potentials measured;
Figure 6 shows the effects of moisturizer on the size and PDI, in which Fig. 6A shows the results of particle sizes measured after the moisturizer was added into the lipid nano-dispersions of this invention, and Fig. 6B shows the results of PDI measured;
Figure 7 shows the results of the mixtures of lipid nano-dispersions containing 0.2g solid lipid with different gel substrates, in which Fig. 7A shows the results of particle sizes measured for the mixtures over 14 days, and Fig. 7B shows the results of PDI measured;
Figure 8 shows the results of the mixtures of lipid nano-dispersions containing 1.0g solid lipid with different gel substrates, in which Fig. 8A shows the results of particle sizes measured for the mixtures over 14 days, and Fig. 8B shows the results of PDI measured;
Figure 9 shows the results of lipid nano-dispersions containing different amount of Poloxamer 188, 5% PEG 400 or 3% Glycerin, in which Fig. 9A shows the results of particle sizes measured for the lipid nano-dispersions over 13 days, and Fig. 9B shows the results of PDI measured;
Figure 10 shows the size distribution of NDEO with 1.0g solid lipid;
Figure 11 shows the TEM photos of NDEO with 1.0g solid lipid, in which the upper one represents LND particles without gel substrate, and the lower one represents LND particles that were mixed with gel substrate;
Figure 12 shows the results of three hemolytic experiments for NDEO with 1.0g SL, 0.6g SL and 0.2g SL, respectively, in which the upper ones show the photos taken at the incubation time of 0.5h, and the lower ones show the photos taken at the incubation time of 3h, and in each photo, the tubes from left to right represent Tube Nos. 1 to 7 as in table 19;
Figure 13 shows the results of Break-up time (BUT) test, in which Fig. 13A shows the results of blank group, Fig. 13B shows the results of XLR group, Fig. 13C shows the results of group A, Fig. 13D shows the results of group B; and Fig. 13E shows the results of group C; Asterisks in Fig. 13 represent that there was statistically significant difference between the BUT values compared with the baseline;
Figure 14 shows the results of Fluorescein staining of cornea, in which Fig. 14A shows the results of blank group, Fig. 14B shows the results of XLR group, Fig. 14C shows the results of group A, Fig. 14D shows the results of group B; and Fig. 14E shows the results of group C; Asterisks in Fig. 14 represent that there was statistically significant difference between the Fluorescein staining scores compared with the baseline; and
Figure 15 shows the results of Historical evaluation, in which Fig. 15A shows the results for Break-up time test, and Fig. 15B shows the results for Fluorescein staining of cornea.

### Detailed Description of the Preferred Embodiments

This invention is now described by way of examples with reference to the figures in the following paragraphs. Objects, features, and aspects of the present invention are disclosed in or are apparent from the following description. It is to be understood by one of ordinary skilled in the art that the present discussion is a description of exemplary embodiments only, and is not intended as limiting the broader aspects of the present invention, which broader aspects are embodied in the exemplary constructions.

It was surprisingly discovered that by using both solid lipid and liquid lipid in a lipid nano-dispersion system, the ophthalmic composition of the present invention can be used for prevention or treatment of dry eye syndrome without containing any active pharmaceutical agent. The ophthalmic composition of the present invention may even manage dry eye syndrome with moderate, to moderate-severe condition.

The ophthalmic composition was further improved by screening the ingredients, and optimizing the formulation and preparation procedures. Furthermore, the medicament effects were proven by animal tests.

In summary, the present invention has achieved the following technical effects:
i) The solid lipid components of the lipid nano-dispersion in the ophthalmic composition of the present invention are selected from the commonly-used eye ointment matrix, which has good biocompatibility and ocular safety;
ii) Both the solid and liquid components are dispersed in nano-scale. Thus, the ophthalmic composition of the present invention preserves the advantages of the eye ointment with reduced eyelid adhesions and blurred vision;
iii) The ophthalmic composition of the present invention is semi-transparent, and therefore could be used during daytime;
iv) The ophthalmic composition of the present invention can be utilized in repairing the tear film, lubricating the eye, reducing tear evaporation and carries sustained release advantages;
v) The ophthalmic composition has a lipid particle size, preferably, equal to or less than 200nm, thus allowing the composition to adhere and retain inside the conjunctival sac for longer duration, and leaving less residue after use;
vi) The gel substrate in the current innovation composition can further extend the retainability of the lipid nano-dispersion in the eye, along with additional lubricating and moisturizing effect;
vii) The ophthalmic composition of the present invention is also a versatile technology platform that can be used for delivering both hydrophobic and hydrophilic medicine to the eye.

### Ophthalmic composition

In one embodiment, the ophthalmic composition of the present invention includes a mixture of lipid nano-dispersion and gel substrate, wherein the lipid nano-dispersion includes a first lipid, a second lipid, and emulsifier; in which the first lipid exists in form of solid lipid as staring material, and the second lipid exists in form of liquid lipid as staring material.

As used herein, term "solid lipid" refers to a lipid in solid form when stored at room temperature, 1 atm.

As used herein, term "liquid lipid" refers to a lipid in liquid form when stored at room temperature, 1 atm.

As used herein, term "room temperature" refers to indoor temperature of from 18 to 25°C.

As used herein, term "lipid nano-dispersion" refers to a dispersion system of solid lipid nanoparticle (SLN). The SLN is typically spherical with an average diameter between 10 to 1000 nanometers. Solid lipid nanoparticles possess a solid lipid core matrix that can solubilize lipophilic molecules. The lipid core is stabilized by surfactants (emulsifiers).

The lipid nano-dispersion is similar to the nanostructured lipid carrier that was developed from SLN. As an alternative carrier to emulsions, polymeric nanoparticles and liposomes, SLN was firstly developed in 1990s and then became a research hot topic for the pharmaceutics scientists. To prepare SLN, highly purified lipids are usually applied in the carrier matrix. However, the drug could be expelled from the SLN because the lipid matrix tends to form a relatively perfect crystal lattice over time during storage. Therefore, liquid lipids are usually added into the formulation to increase the imperfection of the matrix, and consequently the stability of the formulation is improved. The nanostructured lipid carrier (NLC) can be used to deliver the lipophilic drug to the eye to improve the bioavailability of the active drug. However, it is not known to the inventors whether there is any report of using the nanostructured lipid carrier matrix to treat the dry eye disease. In addition, the stability of the nanostructured lipid carriers in prior art that were formulated by using either pure solid lipid(s) or pure liquid lipid(s) is poor when used as drug carrier.

The lipid nano-dispersion is an aqueous dispersion that contains nanoscale-dispersed lipids. Compared to NLC, lipid nano-dispersion has a higher concentration of liquid lipids. Therefore the solid lipids maybe entrapped in the liquid lipids. And the carrier is also stabilized by emulsifiers.

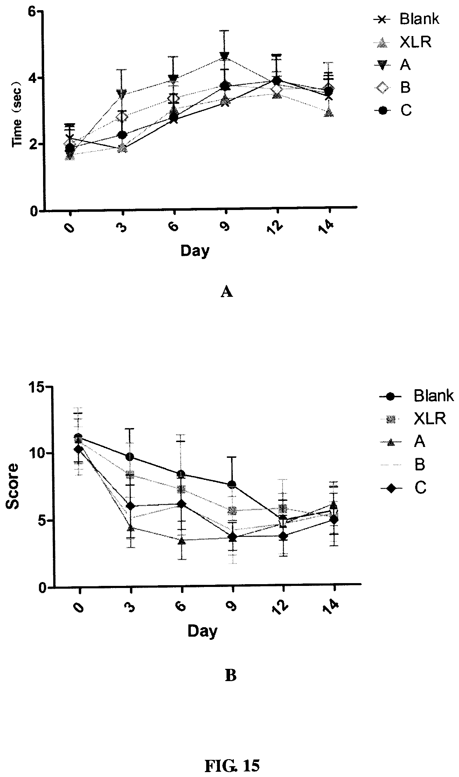

Lipid nano-dispersion is different from microemulsions, for which, microemulsions are clear, thermodynamically stable, isotropic liquid mixtures of oil, water and surfactant, frequently in combination with a cosurfactant. The aqueous phase of microemulsions may contain salt(s) and/or other ingredients, and the "oil" may actually be a complex mixture of different hydrocarbons and olefins. By contrast, the lipid phase of the lipid nano-dispersion contains solid lipid, while that of microemulsions contain only liquid oil. They have different lubricating and sustained release effect. More specifically, by introducing solid lipids, the lipid nano-dispersion has an improved lubricating effect compared to microemulsions. Efficacy study of the present invention also shows that the effect of treatment on dry eye syndrome depends on the content of solid lipids, as when the lipid nano-dispersion having a higher content of solid lipids was administered, the resulting break-up time was longer (see Fig. 15A), and the fluorescein staining score was lower (see Fig. 15B). In addition, the lipid nano-dispersion having a higher content of solid lipids has better sustained release effect compared to microemulsions.

In one preferred embodiment, the solid lipid includes yellow Vaseline and wool fat. Preferably, the yellow Vaseline and the wool fat are at weight ratio of (4-10) : 1; more preferably, the yellow Vaseline and the wool fat are at weight ratio of 8 : 1.

It is discovered that it is highly advantageous to use both Yellow Vaseline and wool fat in the formulation of the lipid nano-dispersion. As far as yellow Vaseline is concerned, it is a semisolid mixture of linear paraffins that are extracted from petroleum. Thus yellow Vaseline could cause crystal imperfection, making the carrier relatively more stable. Yellow Vaseline and wool fat have been used as the matrix of eye ointment for a long time and therefore they are very safe in clinic. However there is no report of using both yellow Vaseline and wool fat as the solid lipids of a lipid nano-dispersion. Considering the safety of the matrix of eye ointment, the superiority of using yellow Vaseline and wool fat is quite significant. Additionally, the joint use of yellow Vaseline and wool fat in the lipid nano-dispersion provides a good ocular lubricant effect, and can also disperse on the surface of tear layer of the eye, and consequently reducing the evaporation of tear. Thus it is advantageous to use both yellow Vaseline and wool fat as the solid lipids of the lipid nano-dispersion.

Preferably, based on the total volume of the ophthalmic composition, the ophthalmic composition includes 0.1% ~ 6% (w/v) of the first lipid; more preferably, the ophthalmic composition includes 0.1% ~ 0.5% (w/v) of the first lipid.

Any commonly-used liquid lipid for intravenous injection or eye applications can be used in the present invention, as long as they are moderate, little or non-irritative to eye, and non-toxic for eye applications. The liquid lipid includes, but not limited, at least one of medium-chain triglycerides, propylene glycol dicaprylate/dicaprate (e.g., MIGLYOL840), naphthenic mineral oil (e.g., CAS NO. 8012951), mineral oil (e.g., CAS NO. 8042475), castor oil, polyoxyethylene castor oil (e.g., CAS NO. 61791126), and polyoxyethylene hydrogenated castor oil (e.g., CAS NO. 61788850).

Preferably, the liquid lipid includes medium-chain triglycerides; more preferably, the medium-chain triglycerides include caprylic/capric triglyceride; further preferably, caprylic/capric triglyceride include at least one of Estasan 3575, Miglyol® 812, and Crodamol GTCC.

Preferably, based on the total volume of the ophthalmic composition, the ophthalmic composition includes 0.1% ~ 10% (w/v) of the second lipid; more preferably, the ophthalmic composition includes 0.25% ~ 8% (w/v) of the second lipid.

The emulsifier may include an oil-phase emulsifier only, with an optional aqueous-phase emulsifier. Preferably, the emulsifier includes at least one oil-phase emulsifier and at least one aqueous-phase emulsifier, as the emulsification effect obtained by using such combination of emulsifiers is more desirable.

Preferably, based on the total volume of the ophthalmic composition, the ophthalmic composition includes 0.1% ~ 6% (w/v) of the at least one oil-phase emulsifier; more preferably, the ophthalmic composition includes 0.2% ~ 3.5% (w/v) of the at least one oil-phase emulsifier.

Preferably, the at least one oil-phase emulsifier includes at least one of Egg yolk phosphatidylcholine (EPC), Soybean phospholipids, TegoCare, Hydrogenated soybean phosphatidylcholine, Myverol, and Span; more preferably, the at least one oil-phase emulsifier includes Egg yolk phosphatidylcholine.

Preferably, based on the total volume of the ophthalmic composition, the ophthalmic composition includes 0.1% ~ 8% (w/v) of the at least one aqueous-phase emulsifier; more preferably, the ophthalmic composition includes 0.25% ~ 6% (w/v) of the at least one aqueous-phase emulsifier. Specifically, when the content of poloxameris is higher, the particle size and PDI became larger, while when the content of poloxamer is lower, the emulsifying effect might be poor, which can be observed and judged by the physical appearance of the ophthalmic composition that it became less transparent.

Preferably, the at least one aqueous-phase emulsifier includes at least one of Poloxamer 188, Tween, sodium cholate, Sodium deoxycholate, Cremphor EL, and Solutol® HS; more preferably, the at least one aqueous-phase emulsifier includes Poloxamer 188.

Preferably, the gel substrate includes a gel-forming agent dispersed in a buffer solution; more preferably, the gel substrate further includes a water-retaining agent.

The water-retaining agent may reduce water evaporation for alleviating eye dryness. The water-retaining agent is optional in the ophthalmic composition.

Preferably, the gel-forming agent includes at least one of polyvinyl pyrrolidone (PVP), polyacrylates, and polysaccharides. Examples of polysaccharides include, but not limited to, hyaluronic acid, chitosan, xanthan gum, gellan gum, amylopectin, and cellulose derivatives. Examples of cellulose derivatives include, but not limited to, methyl cellulose (MC), hydroxy propyl methyl cellulose (HPMC), and carboxymethyl cellulose sodium (CMC-Na).

More preferably, the gel-forming agent includes polyvinyl pyrrolidone. Polyvinyl pyrrolidone is more preferred than hydroxy propyl methyl cellulose, as the ophthalmic composition containing polyvinyl pyrrolidone has a less Polydispersity Index (PDI) value than one containg hydroxy propyl methyl cellulose. Carbopol is not desirable in the present invention, as a formulation containing carbopol was found to be less stable.

Preferably, based on the total volume of the ophthalmic composition, the ophthalmic composition includes 0.2% ~ 3% (w/v) of the gel-forming agent; more preferably, the ophthalmic composition includes 1% ~ 1.8% (w/v) of the gel-forming agent.

Preferably, the water-retaining agent (also called moisture) includes at least one of polyethylene glycol (PEG), glycerin, hyaluronic acid, chitosan, chondroitin sulfate, and sodium hyaluronate; more preferably, the water-retaining agent includes glycerin.

Chitosan is a cationic, biodegradable, biocompatible and non-toxic polymer. It possesses antimicrobial and wound healing properties. Moreover, chitosan exhibits a pseudoplastic and viscoelastic behavior. It can be used as gel-forming agent, and ocular retaining agent, which is an agent that can extend the duration of drug and carrier in the eye, as well as water-retaining agent.

Hyaluronic acid can be used as both gel-forming agent and moisturizer. When hyaluronic acid is used as gel-forming agent, it could retain the water. And hyaluronic acid may also benefit the therapy of dry eye disease as an active agent.

For eye applications, hyaluronic acid and sodium hyaluronate can improve bioavailability of drugs, and have functions of moisturizing and lubricating, anti-inflammation, and repairing, which would be an ideal water-retaining agent if cost is not the only consideration. Chondroitin sulfate can be used to repair cornea damage, improve the absorption of extravasate, and reduce inflammation. However, comparing to glycerin, chondroitin sulfate is less desirable due to costs.

Preferably, based on the total volume of the ophthalmic composition, the ophthalmic composition includes 0.1 % ~ 5% (w/v) of the water-retaining agent; more preferably, the ophthalmic composition includes 0.1% ~ 3% (w/v) of the water-retaining agent.

Preferably, the buffer solution includes at least one of borate saline buffer solution, phosphate buffer solution, Gifford's buffer solution (i.e., a buffer solution that can be prepared by mixing an acidic solution containing 12.4g/L H₃BO₃ and 7.4g/L KCI with a basic solution containing 21.2g/L Na₂CO₃ in different ratios to obtain a pH in a range of 4.66 ~ 8.47), sodium acetate-boric acid buffer solution, Atking and parting buffer solution (i.e., a buffer solution that can be prepared by mixing an acidic solution containing 12.4g/L H₃BO₃ and 7.5g/L NaCl with a basic solution containing 21.2g/L Na₂CO₃ in different ratios to obtain a pH in a range of 7.6 ~ 11.0) and Feldman buffer solution (i.e., a buffer solution that can be prepared by mixing an acidic solution containing 12.4g/L H₃BO₃ and about 2.9g/L NaCl with a basic solution containing about 19.1g/L Na₂[B₄O₅(OH)₄]·8H₂O in different ratios to obtain a pH in a range of 6.0 ~ 8.2). Preferably, the concentration of the buffer agent in the buffer solution is in a range of 1v0mM ~ 300mM; more preferably, the concentration of the buffer agent in the buffer solution is in a range of 20mM ~ 200mM.

More preferably, the buffer solution includes borate saline buffer solution. Borate saline buffer solution is a commonly-used pH regulator for eye applications, which is non-toxic and non-irritative to eye, and safe to use.

Preferably, the ophthalmic composition has a pH in a range of 4~9; more preferably, the ophthalmic composition has a pH in a range of 6~9; further preferably, the ophthalmic composition has a pH in a range of 6~8. When pH is in a range of 6~8, the ophthalmic composition is less irritative to eye.

In one preferred embodiment, based on the total volume of the ophthalmic composition, the ophthalmic composition includes:
0.1% ~ 6% (w/v) of the solid lipid, in which the solid lipid includes yellow Vaseline and wool fat at weight ratio of (4-10) : 1;
0.1% ~ 10% (w/v) of the liquid lipid;
0.1% ~ 6% (w/v) of the at least one oil-phase emulsifier;
0.1% ~ 8% (w/v) of the at least one aqueous-phase emulsifier;
0.2% ~ 3% (w/v) of the gel-forming agent;
0.1 % ~ 5% (w/v) of the water-retaining agent;
10mM ~ 300mM of the buffer solution, pH 4~9.

In another preferred embodiment, based on the total volume of the ophthalmic composition, the ophthalmic composition includes:
0.1% ~ 0.5% (w/v) of the solid lipid, in which the solid lipid includes yellow Vaseline and wool fat at weight ratio of (4-10) : 1;
0.25% ~ 8% (w/v) of the liquid lipid;
0.2% ~ 3.5% (w/v) of the at least one oil-phase emulsifier;
0.25% ~ 6% (w/v) of the at least one aqueous-phase emulsifier;
1% ~ 1.8% (w/v) of the gel-forming agent;
0.1 % ~ 3% (w/v) of the water-retaining agent;
20mM ~ 200mM of the buffer solution, pH 6~9.

In yet another preferred embodiment, based on the total volume of the ophthalmic composition, the ophthalmic composition includes:
0.1% ~ 0.5% (w/v) of the solid lipid, in which the solid lipid includes yellow Vaseline and wool fat at weight ratio of 8 : 1;
2 % (w/v) of the liquid lipid;
1.25% (w/v) of the at least one oil-phase emulsifier;
0.25% (w/v) of the at least one aqueous-phase emulsifier;
1% (w/v) of the gel-forming agent;
0.3% (w/v) of the water-retaining agent;
50mM of the buffer solution, pH 6~8.

Preferably, the ophthalmic composition has a lipid particle size in a range of 20 ~ 500 nm; more preferably, the ophthalmic composition has a lipid particle size in a range of 50 ~ 400 nm; further preferably, the ophthalmic composition has a lipid particle size in a range of 50 ~ 200 nm.

Lipid particle size may be measured by dynamic light scattering theory, as described in Rui Liu, Zhidong Liu, Chengui Zhang, Boli Zhang, Nanostructured Lipid Carriers As Novel Ophthalmic Delivery System for Mangiferin: Improving In Vivo Ocular Bioavailability. Journal of pharmaceutical science, VOL.101, NO.10, OCTOBER 2012, P3833-3844.

If the lipid particle size is relatively large, the particles tend to aggregate and will have shorter retention in eye, such that, although a single particle may carry more drug, the lipid carrier would be removed before the drug is thoroughly released.

Preferably, the ophthalmic composition has a Polydispersity Index (PDI) less than 0.5; more preferably, the ophthalmic composition has a Polydispersity Index less than 0.4; further preferably, the ophthalmic composition has a Polydispersity Index less than 0.3.

In physical and organic chemistry, dispersity is a measure of the heterogeneity of sizes of molecules or particles in a mixture. A collection of objects is considered monodisperse if the objects have the same size, shape, or mass. A sample of objects that have an inconsistent size, shape and mass distribution is called polydisperse. The polydispersity index (PDI) or heterogeneity index, or simply dispersity (Ð), is a measure of the distribution of molecular mass in a given polymer sample. PDI can be measured as described in Peter Atkins and Julio De Paula, Atkins' Physical Chemistry, 9th edition (Oxford University Press, 2010, ISBN 978-0-19-954337-3). If PDI is relatively small, it can be concluded that the obtained nano-product has a homogeneous particle size and good stability, for which the quality cab be controlled more easily.

Preferably, the ophthalmic composition has a zeta potential in a range of ± 5~50 mV; more preferably, the ophthalmic composition has a zeta potential in a range of ± 10~40 mV; further preferably, the ophthalmic composition has a zeta potential in a range of ± 20~30 mV. "Zeta potential" is known in the field of the present invention, as described in, for example, Physical Chemistry, Editor in Chief: Xinpu hou, China Medical Science Press, 1st edition, 1996, P264, and Libo Wu, Jian Zhang, Wiwik Watanable, Physical and chemical stability of drug nanoparticles. Advanced Drug Delivery Reviews.2011,volume 63, P456-469. From a theoretical viewpoint, zeta potential is electric potential in the interfacial double layer (DL) at the location of the slipping plane versus a point in the bulk fluid away from the interface. The significance of zeta potential is that its value can be related to the stability of colloidal dispersions. The zeta potential indicates the degree of repulsion between adjacent, similarly charged particles in a dispersion. For molecules and particles that are small enough, a high zeta potential will confer stability, i.e., the solution or dispersion will resist aggregation. When the potential is low, attraction exceeds repulsion and the dispersion will break and flocculate. So, colloids with high zeta potential (negative or positive, e.g., ± 50 mV for the present invention) are electrically stabilized, while colloids with low zeta potentials tend to coagulate or flocculate (e.g., ± 5 mV for the present invention).

Preferably, the ophthalmic composition includes at least one drug; more preferably, the at least one drug includes at least one of Cyclosporine, Tacrolimus, Flurbiprofen, Triamcinolone Acetonide, Tobramycin, Difluprednate, and Latanoprost. Cyclosporine and Tacrolimus can be used to treat dry eye disease, Flurbiprofen can be used to treat ocular inflammation and cystoid macular edema, Triamcinolone Acetonide can be used to treat retinal vasculature disease and uveitis. Tobramycin can be used to treat pseudomonal keratitis. Difluprednate can be used to treat eye inflammation. Latanoprost can be used to treat open-angle glaucoma or ocular hypertension.

### The method for preparing the ophthalmic composition

In one embodiment, the method of the present invention for preparing the ophthalmic composition includes:
I) independently preparing a lipid nano-dispersion and a gel substrate,
   wherein the preparation of the lipid nano-dispersion includes:
   i) mixing an oil phase dispersion containing a solid lipid, a liquid lipid, an oil-phase emulsifier dispersed in an organic solvent, preferably at a temperature in a range of 60 ~ 90°C, with water or an aqueous phase solution containing a aqueous-phase emulsifier dissolved in water, preferably at a temperature in a range of 60 ~ 90°C, to obtain a preemulsion, preferably for 3-10 min;
   ii) homogenizing the preemulsion, preferably under a pressure in a range of 500 ~ 1500 bar for 6-20 cycles;
   iii) removing the organic solvent from the homogenized preemulsion by evaporation; and
   iv) solidifying the preemulsion, preferably at a temperature in a range of 15 ~ 30°C for 10-60 min and ice bathed for 5-30 min, to obtain the lipid nano-dispersion; and
      wherein the preparation of the gel substrate includes: dispersing a gel-forming agent and a water-retaining agent in a buffer solution; and
II) mixing the lipid nano-dispersion and gel substrate.

In another embodiment, the method of the present invention for preparing the ophthalmic composition includes:
I) independently preparing a lipid nano-dispersion and a gel substrate,
   wherein the preparation of the lipid nano-dispersion includes:
   i) mixing an oil phase dispersion containing a solid lipid, a liquid lipid, an oil-phase emulsifier dispersed in an organic solvent, preferably at a temperature in a range of 60 ~ 90°C, with water or an aqueous phase solution containing a aqueous-phase emulsifier dissolved in water, preferably at a temperature in a range of 60 ~ 90°C, to obtain a preemulsion, preferably for 5 min;
   ii) homogenizing the preemulsion, preferably under a pressure in a range of 500 ~ 1500 bar for 12 cycles;
   iii) removing the organic solvent from the homogenized preemulsion by evaporation; and
   iv) solidifying the preemulsion, preferably at a temperature in a range of 15 ~ 30°C for 15 min and ice bathed for 10 min, to obtain the lipid nano-dispersion; and
      wherein the preparation of the gel substrate includes: dispersing a gel-forming agent and a water-retaining agent in a buffer solution; and
II) mixing the lipid nano-dispersion and gel substrate.

### Use of the ophthalmic composition

The present invention provides the ophthalmic composition for use in preventing or treating dry eye syndrome, ocular inflammation, cystoid macular edema, or Glaucoma. Specifically, the ophthalmic composition can manage dry eye syndrome with moderate, to moderate severe condition.

The ophthalmic composition according to the present invention can be used for prevention or treatment of dry eye syndrome, ocular inflammation, cystoid macular edema, or Glaucoma.

### Method of treatment of disease

The present invention provides the ophthalmic composition according to the present invention for use in treating a subject having or at risk of developing dry eye syndrome, ocular inflammation, cystoid macular edema, or Glaucoma, including the step of administering to the subject an effective amount of the ophthalmic composition.

The effective amount of the ophthalmic composition for a particular individual depends on the severity of the condition of the individual, the type of formulation being applied, the frequency of administration, and the duration of treatment.

### EXAMPLES

### Materials and Methods

Lipid particle size and Polydispersity Index were measured using Zetasizer Nano-ZS, Malvern Instruments Ltd. Each sample was diluted until having faint opalescence, and measured at a scatter angle of 90°, and at the temperature of 25°C±0.2°C, for three times.

Zeta potential was measured using Zetasizer Nano-ZS, Malvern Instruments Ltd. Each sample was diluted until having faint opalescence, and at the temperature of 25°C±0.2°C, for three times.

Osmotic pressure was measured using Osmomat® 030 Automatic Cryoscopic Osmometer. To determine the osmotic pressure, 50 µL sample were added to the eppendorf tube. By the means of crioscopic method, the osmotic pressure was determined for three times.

### Hemolytic experiment:

To evaluate the safety of the formulations, hemolytic experiment was carried out. Twenty milliliter of blood was obtained using a 50ml syringe stabbing into the heart of a New Zealand albino rabbit obtained from Experimental Animal Center of School of Pharmacy, Fudan University, P. R. China. Fibrinogen was removed right away after the blood was extracted from the rabbit heart by quickly stirring the blood with a glass rod. The blood without fibrinogen was washed with excessive 0.9% (w/v) NaCl solution and centrifuged at 1500rpm for 15min for several times to obtain erythrocyte. Thereafter 2ml erythrocyte was diluted to 100ml with 0.9% NaCl solution to obtain 2% (v/v) erythrocyte suspension for hemolytic experiment. For each formulation, seven tubes were prepared and five of them were used, then one of the rest two tubes was used as negative control and the last one was used as positive control. After the erythrocyte suspension was mixed with the formulation or 0.9% NaCl solution or distilled water, the tubes were water bathed at 37°C for up to 3 hours.

### Animal test:

In order to evaluate the therapy effectiveness of the formulations, dry eye disease model that was induced by the preservative benzalkonium chloride (BAC) was applied. Specifically, 60 male BALB/c mice which were about 6-8 weeks old and had the weight of 18-20g were obtained from the Experimental Animal Center of Eye and ENT Hospital, Fudan University, P. R. China. Fifteen mice were excluded after walleyes were founded using slit lamp. BAC in concentration of 0.3% (w/v) was delivered to the eyes of mice twice a day for two weeks. And finally, 41 qualified mice were left for the experiment. The mice were randomly divided into 5 groups. There were 6 mice for blank group, 9 mice for Dextran 70 and glycerol eye drops group (XLR group), 9 mice for group A, 9 mice for group B and 8 mice for group C. Another 12 qualified were used to back up the blank group and to provide normal control for historical evaluation. Each mouse was administered with one drop of the sample for each group, three times a day, over 14 days.

The break-up time (BUT) test is determined by instilling fluorescein in the inferior cul-de-sac and then evaluating the stability of the pre-corneal tear film after 1 to 2 minutes, using a broad beam of the slit lamp with a blue filter. The break-up time is the time that elapses from the last blink to the first appearance of a dark spot in the fluorescein-stained film. The test was carried out at 0, 3, 6, 9, 12, 14 days during the period of administration as described above.

Corneal fluorescein staining was carried out at 0, 3, 6, 9, 12, 14 days during the period of administration as described above. Two minutes after 2 µl of 1% fluorescein sodium was dropped in to the conjunctival sac, the cornea surface was examined and graded under a slit lamp microscope with cobalt blue filter. The cornea and conjunctiva was fixed in 10% formalin. After the specimens were embedded in paraffin, cross-sectioned, and stained with hematoxylin-eosin, the morphology of cornea and conjunctival epithelium and the number of goblet cells in superior and inferior were assessed under microscope by two independent masked observers.

### Example1: Single factor experiment

In order to understand how the ingredients will affect the particle size and PDI of the lipid nano-dispersion, single factor experiment was carried out. The concentration of solid lipids (Yellow Vaseline : Wool fat = 8:1, w/w), liquid lipid (MCT), surfactants (EPC, Poloxamer 188) applied in the formulation was studied relying on a basic formulation.

### Preparation method:

High pressure homogenization was applied for preparation of the lipid nano-dispersion. First of all, solid lipids composing Yellow Vaseline and wool fat (8:1, w/w), liquid lipid (MCT) and EPC were dissolved in 20 ml ethanol at 75°C as oil phase. Then 100 ml water with 0.5% Poloxamer 188 was prepared at 75°C as water phase. And afterwards the oil phase was poured slowly into the water phase under the condition of 1000rpm stirring using IKA® WERKE. The pre-emulsion was obtained after 5min continuous stirring. Subsequently the nanoemulsion was produced by homogenizing the pre-emulsion using AST homogenizer (AH100D, ATS Engineering Inc.). And then the nanoemulsion was water bathed under the condition of 1000rpm stirring at 75°C for 45min to evaporate ethanol. At last the nanoemulsion was cooled down at room temperature for 15min and ice bathed for 10min to solidify the lipid before the lipid nano-dispersion was finally produced.

As far as the formulation of single factor experiments, lipid nano-dispersion was not mixed with gel substrate.

### Formulations:

**Table 1 Basic formulation of lipid nano-dispersion**

| Excipients as staring material | Ratio (%, w/v) |
|---|---|
| Solid lipids | 1 |
| MCT | 4 |
| EPC | 2.5 |
| Poloxamer 188 | 4 |
| Water | 100 |

**Table 2. Study on the change of solid lipids as staring material**

| Excipients as staring material | Lipid nano-dispersion | | | |
|---|---|---|---|---|
| | A | B | C | D |
| Solid lipid (g) | 1 | 2 | 3 | 4 |
| MCT (g) | 4 | 4 | 4 | 4 |
| EPC (g) | 2.5 | 2.5 | 2.5 | 2.5 |
| Poloxamer 188 (g) | 4 | 4 | 4 | 4 |
| Water (ml) | 100 | 100 | 100 | 100 |

**Table 3. Study on the change of MCT**

| Excipients as staring material | Lipid nano-dispersion | | | |
|---|---|---|---|---|
| | E | A | F | G |
| Solid lipid (g) | 1 | 1 | 1 | 1 |
| MCT (g) | 2 | 4 | 6 | 8 |
| EPC (g) | 2.5 | 2.5 | 2.5 | 2.5 |
| Poloxamer 188 (g) | 4 | 4 | 4 | 4 |
| Water (ml) | 100 | 100 | 100 | 100 |

**Table 4. Study on the change of EPC**

| Excipients as staring material | Lipid nano-dispersion | | | |
|---|---|---|---|---|
| | H | I | A | J |
| Solid lipid (g) | 1 | 1 | 1 | 1 |
| MCT (g) | 4 | 4 | 4 | 4 |
| EPC (g) | 0.5 | 1.5 | 2.5 | 3.5 |
| Poloxamer 188 (g) | 4 | 4 | 4 | 4 |
| Water (ml) | 100 | 100 | 100 | 100 |

**Table 5. Study on the change of Poloxamer 188**

| Excipients as staring material | Lipid nano-dispersion | | | |
|---|---|---|---|---|
| | K | L | A | M |
| Solid lipid (g) | 1 | 1 | 1 | 1 |
| MCT (g) | 4 | 4 | 4 | 4 |
| EPC (g) | 2.5 | 2.5 | 2.5 | 2.5 |
| Poloxamer 188 (g) | 0.5 | 2 | 4 | 6 |
| Water (ml) | 100 | 100 | 100 | 100 |

### Results:

### Particle size and PDI:

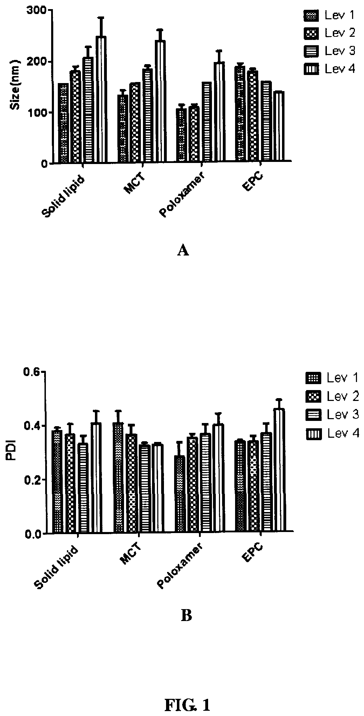

Particle size and PDI were measured to characterize the lipid nano-dispersion. Lev1, Lev2, Lev3, and Lev4 represent the four levels of the increased concentration of different excipients as used in each of Table 2-5. As seen in Fig. 1A, the result showed that the particle size became larger and larger when the concentration of solid lipids, MCT and Poloxamer 188 increased. However, the particle size became smaller and smaller when the concentration of EPC increased. As seen in Fig.1B, when the concentration of Poloxamer188 and EPC increased, the PDI became larger and larger. However, when the concentration of MCT increased the PDI became smaller. In addition, when the concentration of solid lipid increased, the PDI became smaller, except that a sudden increase of PDI was observed when the concentration of solid lipids exceeded 3%.

### Stability study:

### Short term stability of lipid nano-dispersions A-M:

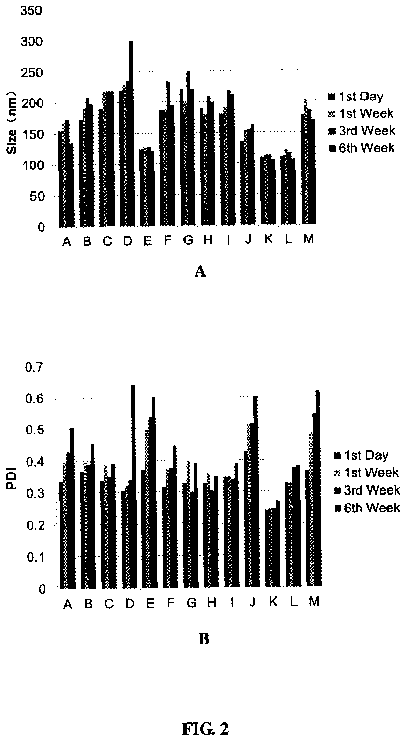

All samples were stored at 4°C, and size and PDI were measured in 1^{st}, 3^{rd} and 6^{th} week. As seen in Fig. 2A and 2B, the result showed that lipid nano-dispersion K was more stable than other lipid nano-dispersions, so lipid nano-dispersion K was selected for the research that followed.

### Long term stability of lipid nano-dispersion K

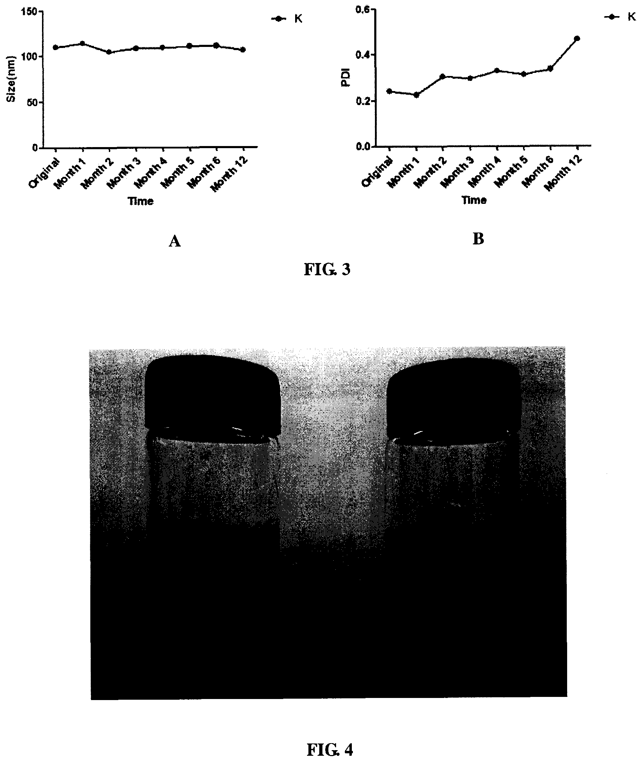

Lipid nano-dispersion K was stored at 4°C for long term stability research. As seen in Fig. 3A and 3B, the result showed that no significant change of particle size was observed when stored at 4°C. Though stored at 4°C for 12 months, the value of PDI was not high.

### Discussion:

Lipid nano-dispersion K was selected from the single factor experiment because of its better stability.
1) For lipid nano-dispersions A-D, the effect of concentration of solid lipids on the particle size and PDI of lipid nano-dispersion was studied. The result displayed that when the particle size of all the lipid nano-dispersions A-D is above 150nm, and the stability is not desirable. High content of solid lipid may cause insufficiently emulsion of the whole dispersion.
2) For lipid nano-dispersions E-G, the effect of concentration of liquid lipid on the particle size and PDI of the lipid nano-dispersion was studied. The result showed that the particle size became bigger and bigger when the concentration of liquid lipid increased. The increase of liquid lipid may also cause insufficient emulsion of the dispersion, which reduces stability.
3) For lipid nano-dispersions H-J, the effect of EPC concentration change on the particle size and PDI of the lipid nano-dispersion was studied. The result showed that the particle size became smaller and smaller when the concentration of EPC increased. When the concentration of EPC was low, poor emulsion occurred; therefore the particle size becomes larger. However when stored for about 6 weeks, all the H-J lipid nano-dispersions showed relatively poor stability and this phenomenon might be caused by the improper ratio of EPC and Poloxamer 188.
4) For lipid nano-dispersions K-M, the particle size became bigger and bigger when the concentration of Poloxamer 188 increases. In a certain concentration range of Poloxamer 188, the particle size did not change significantly. However, when the concentration of Poloxamer 188 is beyond the range, the particle size of lipid nano-dispersion increased significantly, and this phenomenon might be caused by the improper ratio of EPC and Poloxamer 188, other than the lipid nano-dispersion K.

### Example 2: Selection of liquid lipid

The properties of the lipid nano-dispersion were compared to choose a better liquid lipid for the final formulation. The lipid nano-dispersion was prepared using the method mentioned above using MCT (lipid nano-dispersion N) or castor oil (lipid nano-dispersion O) with the following formulations:

| Excipients as staring material | Lipid nano-dispersion | |
|---|---|---|
| | N | O |
| Solid lipid (g) | 1 | 1 |
| MCT (g) | 4 | 0 |
| Castor oil (g) | 0 | 4 |
| EPC (g) | 2.5 | 2.5 |
| Poloxamer 188 (g) | 0.5 | 0.5 |
| Water (ml) | 100 | 100 |

### Result:

**Table 6. Properties of lipid nano-dispersions N and O**

| | N | O |
|---|---|---|
| Size (nm) | 117.8 | 196.8 |
| PDI | 0.249 | 0.251 |
| Zeta potential (mV) | -25.1 | -14.1 |

The data in table 6 showed that the formulation prepared using castor oil had much bigger size than the formulation prepared using MCT. So lipid nano-dispersion O may be less desirable than lipid nano-dispersion N. The |zeta potential| of lipid nano-dispersion N is much bigger than lipid nano-dispersion O. And therefore lipid nano-dispersion N would normally have a better stability compared to lipid nano-dispersion O. As shown in Fig. 4, lipid nano-dispersion N (left bottle) had a more transparent appearance than lipid nano-dispersion O (right bottle). And it is important to have a transparent appearance if the dispersion is to be used in eye drop.

### Example 3: Optimization of the preparation method

Optimization of the preparation method was carried out using the formulation of lipid nano-dispersion A and preparation method as described in Example 1, except that the parameters were varied as shown in table 7.

**Table 7. Parameters used for optimization of the preparation method**

| | Homogenization pressure (bar) | Homogenization cycles | Solidifying strategy |
|---|---|---|---|
| Basic parameter | 1000 | 12 (5min) | Solidify at RT for 15min and then ice bathed for 10min |
| Optimization of homogenization pressure | 800 | * | * |
| | 1000 | * | * |
| | 1200 | * | * |
| Optimization of homogenization cycles | * | 7 (3min) | * |
| | * | 12 (5min) | * |
| | * | 17 (7min) | * |
| | * | 22 (9min) | * |
| Optimization of solidifying strategy | * | * | Solidify at RT for 15min and then ice bathed for 10min |
| | * | * | Ice bathed for 10min |

| | | | |
|---|---|---|---|
| "*" represents that same parameter was used as in Basic parameter. | | | |

### Results:

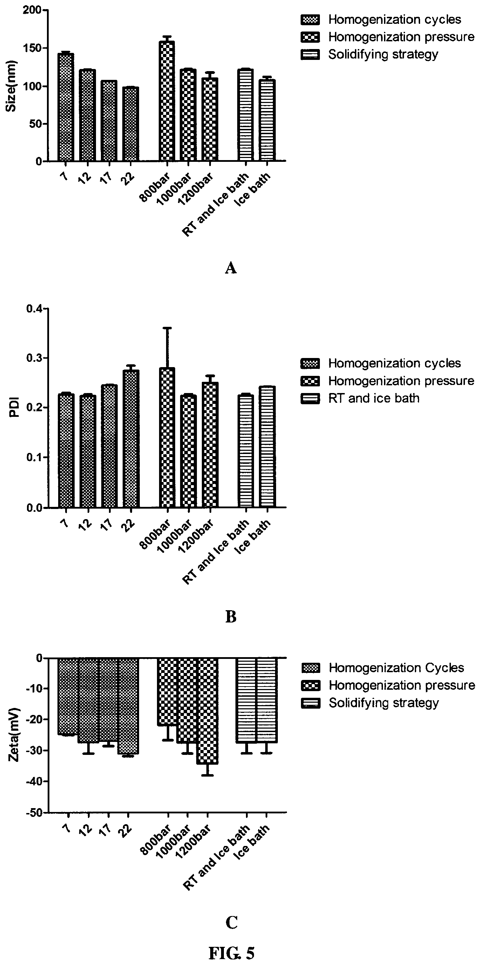

Fig. 5A shows that when the cycles and pressure increased in the homogenization process, the particle size became smaller. In addition, when the nanoemulsion was solidified at the room temperature for 15min followed by ice bath for 10min, formulation with larger particle size was obtained. Fig. 5B shows that when the cycles of homogenization increased, the PDI became bigger. In addition, when the pressure of homogenization was set at 1000bar, the smallest PDI was obtained. Moreover, the nanoemulsion solidified at the room temperature for 15min followed by ice bath for 10min has a smaller PDI. Fig. 5C shows how the zeta potential changes with the preparation parameters, in which the zeta potential became larger when either the cycles or pressure increased in the homogenization process, (except for 17 cycles), but was not significantly changed for different solidifying strategy .

As smaller PDI, larger zeta potential and a particles size around 100nm are preferred, the method that uses 12 cycles of 1000bar homogenization was chosen to prepare the nanoemulsion and the acquired nanoemulsion can then be solidified at RT for 15min before ice bathed for 10min.

### Example 4: Selection of water-retaining agent

In order to reduce the excessive evaporation of tears, two kinds of water-retaining agents (PEG400 and glycerin) were investigated. According to FDA standard (USA), 5% PEG400 or 3% glycerin was used. The particle size, PDI and Zeta potential of lipid nano-dispersion were determined after the water-retaining agent was incorporated into the formulation. And the test of irritation to the eyes for two mixtures 1 and 2, each using PEG or glycerin as the water-retaining agent, respectively, was also carried out. Male New Zealand albino rabbits weighed 2-2.5kg were obtained from the Experimental Animal Center of Eye and ENT Hospital, Fudan University, P. R. China. Before the mixtures 1 and 2 were delivered to the eyes of rabbit, the times of eye blink in 10 min was recorded as control. The times of eye blinks when 40 µl formulation with PEG400 or glycerin was delivered to the eyes of rabbit was recorded for mixtures 1 and 2, respectively. For the control group and each mixture, three rabbits were used, respectively.

### Preparation method:

After the lipid nano-dispersion was prepared as described in Example 1, the PEG400 or glycerin were added thereinto, in an amount as shown in table 8, respectively.

**Table 8. Formulations of mixtures 1 and 2**

| Excipients | Mixture 1 | Mixture 2 |
|---|---|---|
| Solid lipids | 1 | 1 |
| MCT | 4 | 4 |
| EPC | 2.5 | 2.5 |
| Poloxamer 188 | 0.5 | 0.5 |
| PEG400 | 5 | 0 |
| Glycerin | 0 | 3 |
| Water | 100 | 100 |

### Results:

**Table 9. Properties of mixtures 1 and 2**

| | pH | Zeta potential (mv) | Osmotic pressure (mOsm) |
|---|---|---|---|
| Mixture 1 | 6.15 | -16 | 965 |
| Mixture 2 | 7.82 | -33.4 | 1032 |

**Table 10. Times of eye blink**

| | Control | | Mixture 1 | | Mixture 2 | |
|---|---|---|---|---|---|---|
| | Left eye | Right eye | Left eye | Right eye | Left eye | Right eye |
| Times of blink | 5 | 3 | 20 | 17 | 13 | 12 |

The data in tables 9 and 10 showed that when glycerin is used, a formulation can be obtained with higher zeta potential, less irritation to the eyes, and higher osmotic pressure. And a higher zeta potential was usually a sign of better stability. Finally, glycerin was used as the water-retaining agent. According to practical need, the concentration of glycerin is preferably at 0.5%.

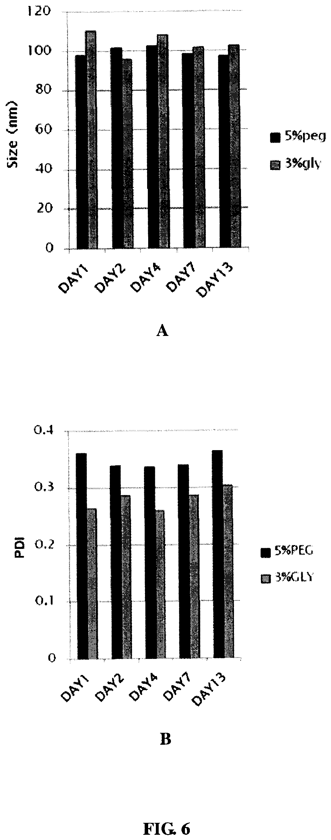

As seen in Fig.6A and Fig.6B, the results showed that when the moisturizers were added into the formulation, there was no significant difference between the two formulations in terms of particle size. And the measurement of particle size showed that the formulation with PEG400 had a smaller particle size, while the formulation with glycerin had a smaller PDI. The stability test showed that when the two formulations were stored at 4°C for 13 days, no significant increase of size and PDI was observed.

### Example 5: First selection of gel substrate:

Gel substrates using Poloxamer, Carbopol or HPMC as gel-forming agent were investigated.

The concentration of Poloxamer 407 was 16%. The concentration of carbopol was 0.1% and 0.2%. The concentration of HPMC was 5%. The viscosities of the gel substrates were determined at 25°C and 34°C, respectively. The viscosities were measured using a rheometer. For the mixtures, the ratio of gel substrates and simulated tear fluid was 40:7(v/v).

### Preparation method:

To prepare the gel substrate of Poloxamer 407, 16g of Poloxamer 407 was added to about 80 ml water. And the mixture was stored at 4°C for one night, then water was added up to 100ml.

To prepare the gel substrate of carbopol, 0.2g and 0.1g carbopol was added to 100ml water, and the mixture was stored at 4°C for one night, then pH of the gel substrate was adjusted to 6 using 1M of NaOH solution.

To prepare the gel substrate of HPMC, 5g of HPMC was added to 80ml of water. And then the mixture was heated to 90°C under continuous stirring. Then water was added up to 100ml.

### Formulations:

**Table 11. Formulation of gel substrate**

| gel-forming agent | content | | | |
|---|---|---|---|---|
| Poloxamer 407 (g) | 16 | 0 | 0 | 0 |
| Carbopol (g) | 0 | 0.2 | 0 | 0 |
| Carbopol (g) | 0 | 0 | 0.1 | 0 |
| HPMC (g) | 0 | 0 | 0 | 5 |
| Water added up to (ml) | 100 | 100 | 100 | 100 |

**Table 12. Formulation of simulated tear fluid**

| Ingredients | Content |
|---|---|
| NaCl | 8.3g |
| CaCl₂ | 0.064g |
| KCl | 1.4g |
| water | 1000 ml |

### Result:

**Table 13. Viscosity test**

| | 16% Poloxamer 407 | | 0.1% Carbopol | | 0.2% Carbopol | | 5% HPMC | |
|---|---|---|---|---|---|---|---|---|
| | Control | Mixture | Control | Mixture | Control | Mixture | Control | Mixture |
| 25°C | 0.12 | 0.045 | 0.13 | 0.031 | 10.06 | 0.019 | 0.96 | 0.61 |
| 34°C | 759.34 | 0.078 | 0.22 | 0.024 | 10.80 | 0.014 | 0.94 | 0.62 |

The result showed that when the gel substrates were mixed with simulated tear fluid, the viscosity decreased significantly for Poloxamer 407 and Carbopol. However the dilution effect of simulated tear fluid to HPMC was not significantly. HPMC has been commonly used as a thickening agent in the solution delivered to the eye. HPMC can also increase the delay the elimination of drugs from the Conjunctival sac and provide lubrication to the eye. In order to avoid foreign body sensation and eyelids adhesions, the final concentration of HPMC is preferably 0.3%.

### Example 6: Second selection of gel substrate

In order to select an optimized gel substrate, lipid nano-dispersion containing different amount of solid lipid was incorporated with three different gel substrates. Then the particle size, PDI and zeta potential of the mixture were determined over 14 days.

### Preparation method:

Two lipid nano-dispersions were prepared as described in Example 1, each containing 1.0g solid lipids or 0.2g solid lipids. And then each lipid nano-dispersion was added to the same volume of three different substrates, respectively.

### Formulations:

**Table 14. Formulation of the lipid nano-dispersion**

| Excipients | lipid nano-dispersion (0.2g solid lipid) | lipid nano-dispersion (1.0g solid lipid) |
|---|---|---|
| Solid lipids | 0.2g | 1.0g |
| MCT | 4g | 4g |
| EPC | 2.5g | 2.5g |
| Poloxamer 188 | 0.5g | 0.5g |
| Water | 100ml | 100ml |

**Table 15. Formulation of the three gel substrates**

| | Substrate A | Substrate B | Substrate C |
|---|---|---|---|
| Glycerin | 1g | 1g | 1g |
| Boric acid | 0.62g | 0.62g | 0.62g |
| HPMC | 0.6g | 0 | 0 |
| Carbopol | 0 | 0.2g | 0 |
| PVP | 0 | 0 | 2g |
| Water | 100ml | 100ml | 100ml |

### Results:

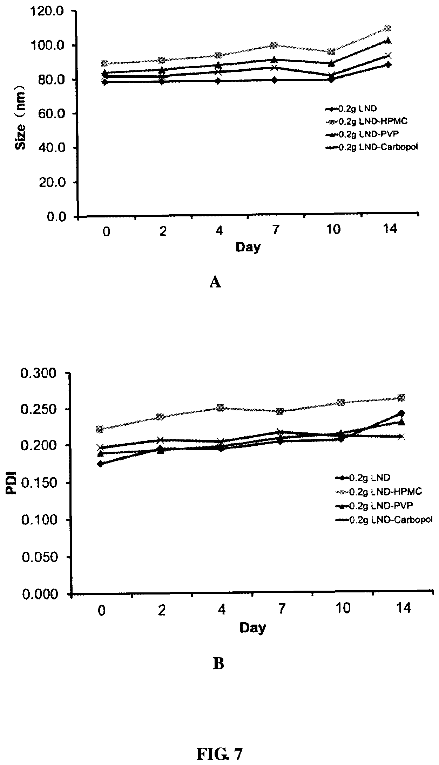

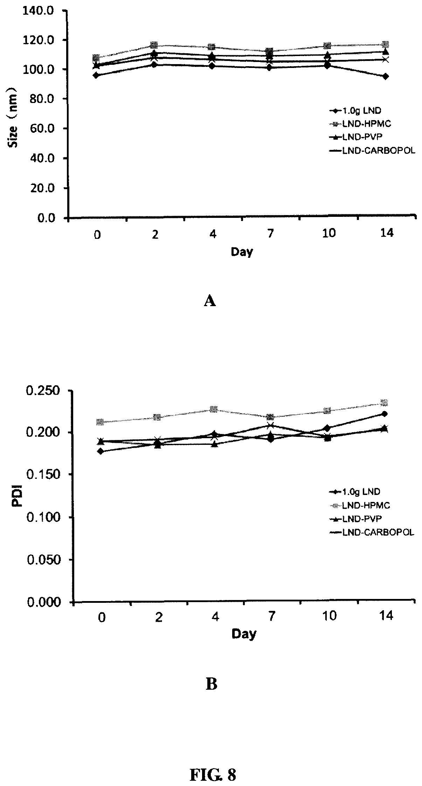

As shown in Figs. 7 and 8, each lipid nano-dispersion mixed with the same volume of gel substrate showed good stability for two weeks. However, the mixtures containing carbopol or HPMC were found to be difficult to filter through the 0.22 µm membrane, and precipitation was also observed for the mixtures containing carbopol. However, mixtures containing HPMC showed larger PDI and particle size than mixtures containing PVP. Therefore PVP was preferably used in the gel substrate.

### Example 7: Storing temperature investigation

To evaluate the stability of lipid nano-dispersion stored at 4°C and 25°C, the lipid nano-dispersion that contains 5% PEG400, 3% glycerin, 0% Poloxamer 188, 0.5% Poloxamer 188, or 2% Poloxamer188 were stored at 4°C and 25°C. The particle size and PDI were measured over 13 days.

### Preparation method:

After the lipid nano-dispersion was prepared as described in Example 1, PEG400 or glycerin were added thereinto, in an amount as shown in table 16, respectively. Specifically, for the lipid nano-dispersion with 0%, 0.5% and 2% Poloxamer 188, the concentration of Poloxamer 188 was adjusted in the aqueous phase before the preparation process started. And for the lipid nano-dispersion with 5% PEG400 and 3% Glycerin, PEG400 and glycerin were added to the formulation after the lipid nano-dispersion was acquired.

### Formulations:

**Table 16. Formulation for storing temperature investigation**

| Ingredients | PEG 5% | Glycerin 3% | Poloxamer 188 0% | Poloxamer 188 0.5% | Poloxamer 188 2% |
|---|---|---|---|---|---|
| Solid lipids (g) | 1 | 1 | 1 | 1 | 1 |
| MCT (g) | 4 | 4 | 4 | 4 | 4 |
| EPC (g) | 2.5 | 2.5 | 2.5 | 2.5 | 2.5 |
| Poloxamer 188 (g) | 0.5 | 0.5 | 0 | 0.5 | 2.0 |
| Water (ml) | 100 | 100 | 100 | 100 | 100 |
| PEG400 (g) | 5 | 0 | 0 | 0 | 0 |
| Glycerin (g) | 0 | 3 | 0 | 0 | 0 |

### Results:

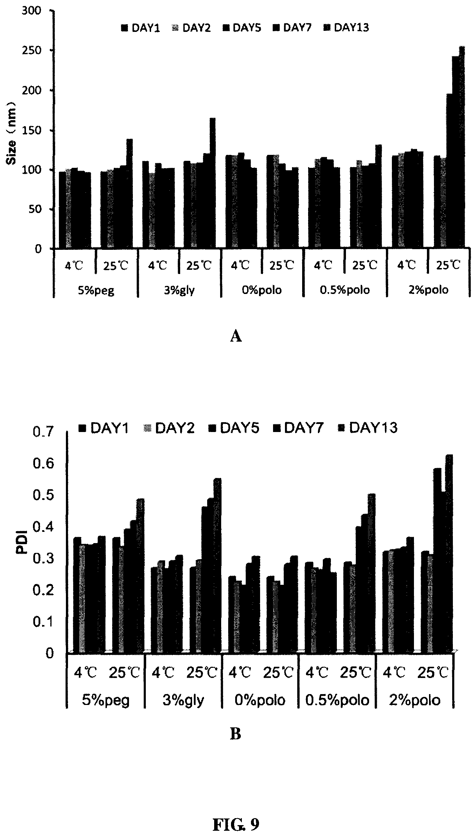

As shown in Fig. 9, lipid nano-dispersion stored at 4°C showed better stability. And the result also showed that when the concentration of Poloxamer 188 increased, the stability of lipid nano-dispersion became poorer.

### Example 8: Investigation of PVP matrix

### Preparation method:

In order to produce lipid nano-dispersion, high pressure homogenization was applied. First of all, 1, 0.6, 0.2 g solid lipids composing of Yellow Vaseline and wool fat (8:1, w/w), 4 g liquid lipid (MCT) and 2.5 g EPC were dissolved in 20 ml ethanol at 75°C as oil phase. Then 100 ml water with 0.5% Poloxamer 188 was prepared at 75°C as water phase. And afterwards the oil phase was poured slowly into the water phase under the condition of 1000rpm stirring using IKA®WERKE. The pre-emulsion was obtained after 5min continuous stirring. Subsequently the nanoemulsion was produced by homogenizing the pre-emulsion using AST homogenizer. And then the nanoemulsion was water bathed under the condition of 1000rpm stirring at 75°C for 45min to evaporate ethanol. At last the nanoemulsion was cooled down at room temperature for 15min and ice bathed for 10min before the lipid nano-dispersion was finally produced.

For the preparation of final formulation named NDEO (i.e., Nanoscale-dispersed eye ointment), lipid nano-dispersion was mixed with the equal volume water containing 0.6% glycerin, 2% PVP, 100mM boric acid and 0.02% sodium hydroxide under 1000rpm continuous stirring condition for 30min using a magnetic stirrer (IKA® Color squid, Germany) at room temperature.

### Formulations:

**Table 17. Formulation of NDEOs**

| Excipients | content | | | function |
|---|---|---|---|---|
| Yellow Vaseline | 0.889g | 0.533g | 0.178g | solid lipid |
| Wool fat | 0.111g | 0.067g | 0.022g | solid lipid |
| Total | 1g | 0.6g | 0.2g | - |
| MCT | 4g | | | liquid lipid |
| EPC | 2.5g | | | emulsifier |
| Poloxamer 188 | 0.5g | | | emulsifier |
| PVP K29/32 | 2g | | | gel-forming agent |
| Glycerin | 0.6g | | | Water-retaining agent |
| Boric acid | 0.62g | | | Buffer agent |
| NaOH | 0.02g | | | pH regulator |
| Water | 200ml | | | solvent |

### Results:

### 1) Characterization:

**Table 18. Properties of NDEOs**

| | Osmotic pressure (mOsm) | pH | Particle size (nm) | PDI | Zeta potential (mv) |
|---|---|---|---|---|---|
| NDEO with 1.0g Solid lipid (SL) | 318 | 7.36 | 94.4±2.3 | 0.212±0.008 | -29.0±0.7 |
| NDEO with 0.6g SL | 315 | 7.36 | 90.8±0.6 | 0.207±0.007 | -27.1±2.0 |
| NDEO with 0.2g SL | 299 | 7.34 | 87.9±0.6 | 0.212±0.010 | -28.8±2.6 |

### 2) TEM, Size distribution:

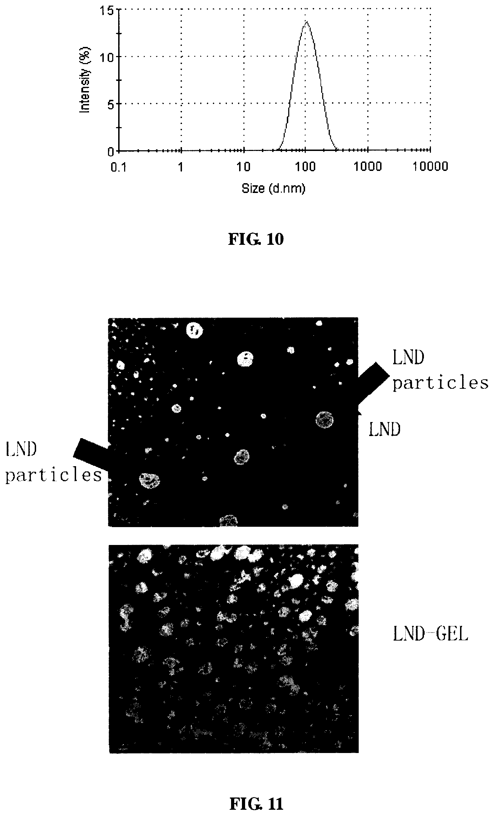

Fig. 10 was export from the Malvern Zetasizer when lipid particle size were measured, and Fig. 11 was obtained using Jeol JEM-1230 electron microscope (Tokyo, Japan).

As shown in Fig. 10, the NDEO with a narrow size distribution was obtained, and the TEM as in Fig. 11 also showed that the lipid particles had spherical shapes.

### 3) Hemolytic experiment:

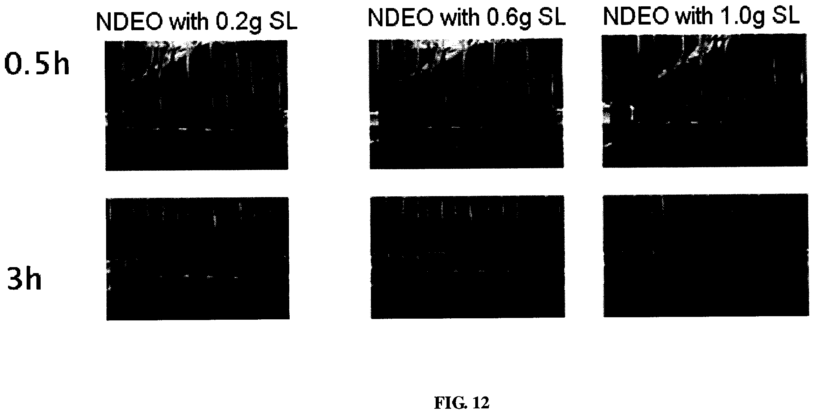

Three hemolytic experiments for NDEO with 1.0g SL, 0.6g SL and 0.2g SL, respectively, were carried out using the mixing ratio as shown in table 19, the result is shown in Fig. 12.

**Table 19. Mixing ratio used in Hemolytic experiment**

| | | | | | | | |
|---|---|---|---|---|---|---|---|
| No. of tube | 1 | 2 | 3 | 4 | 5 | 6 | 7 |
| 2% erythrocyte (ml) | 2.5 | 2.5 | 2.5 | 2.5 | 2.5 | 2.5 | 2.5 |
| 0.9% NaCl solution (ml) | 2.0 | 2.1 | 2.2 | 2.3 | 2.4 | 2.5 | 0 |
| Water (ml) | 0 | 0 | 0 | 0 | 0 | 0 | 2.5 |
| NDEO (ml) | 0.5 | 0.4 | 0.3 | 0.2 | 0.1 | 0 | 0 |

As shown in Fig. 12, no hemolysis phenomenon was observed for NDEO with 1.0g SL, 0.6g SL and 0.2g SL, respectively. So the formulation of NDEO would not cause irritation to the eye.

### 4) Efficacy study:

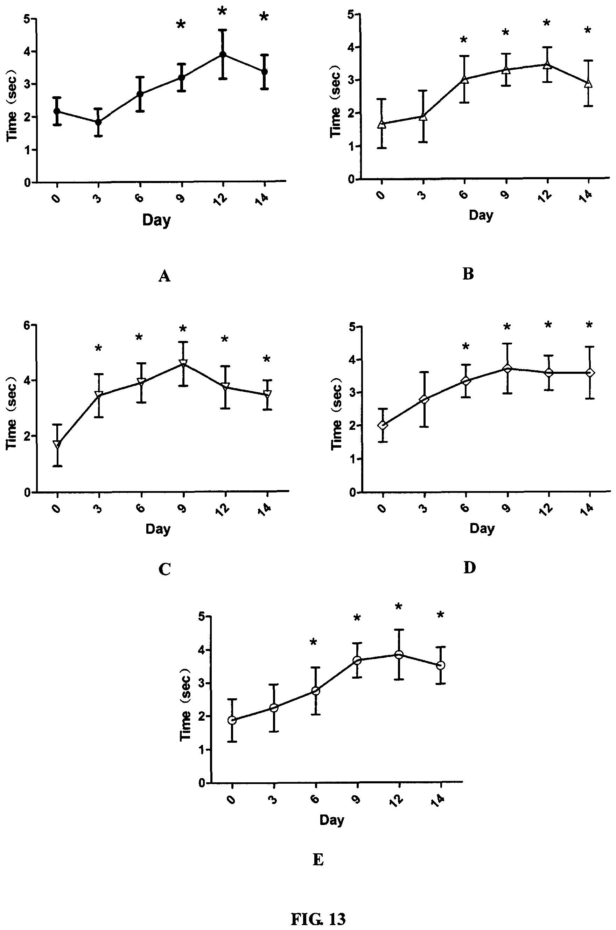

In Figs. 13-15, Blank represents the group without receiving any treatment; group XLR represent the group treated with dextran 70 and glycerol eye drops (Tears Naturale® Forte (Alcon, Inc., USA)); group A represents NDEO with 1.0g solid lipids; group B represents NDEO with 0.6g solid lipids; group C represents NDEO with 0.2g solid lipids.

As show in Figs. 13-15, the therapy effectiveness of NDEOs was better than the dextran 70 and glycerol eye drops. And the NDEO with 1.0g solid lipids was the best one.

Moreover, as seen in Fig.13A, the result showed that for the blank group, the significant improvement of BUT was observed on 9th day. As seen in Fig.13B, for the group treated with dextran 70 and glycerol eye drops, significant improvement was observed on 6th day. As seen in Fig.13C, for the group treated with NDEO with 1.0g solid lipids (group A), significant improvement was observed on 3rd day. As seen in Figs.13D and 13E, significant improvement was observed on 6th day for each of groups B and C.

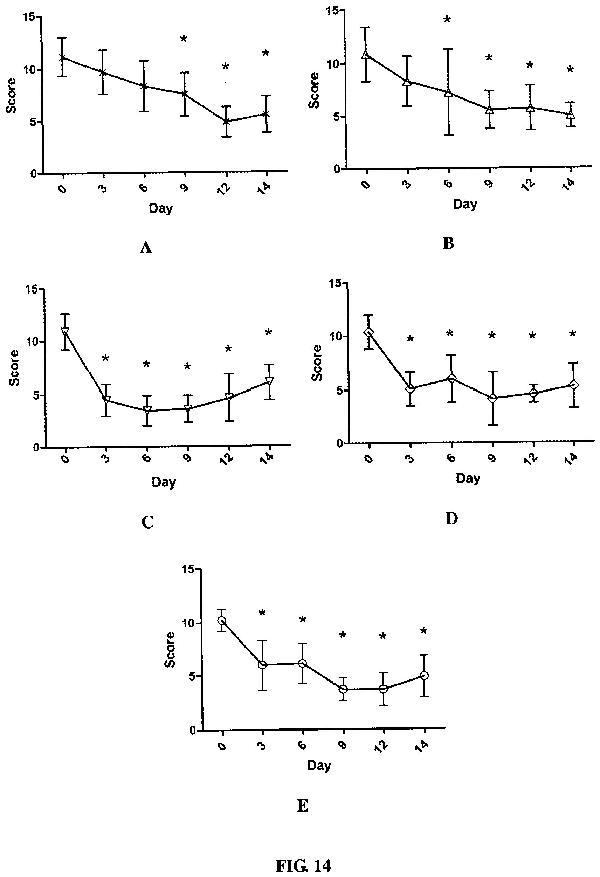

As seen in Fig.14A, the result showed that a significant decrease of Fluorescein staining score was observed for blank group on 9th day. As seen in Fig.14B, for the XLR group, significant decrease of Fluorescein staining score was observed on 6th day. As seen in Fig.14C, 14D and 14E, for each of groups A, B and C, significant decrease of Fluorescein staining score was observed on 3rd day.

All the results of BUT and Fluorescein staining was overlay in Fig.15, which shows that effect of treatment on dry eye syndrome depends on the content of solid lipids, as when the lipid nano-dispersion having a higher content of solid lipids was administered, the resulting break-up time was longer (see Fig. 15A), and the fluorescein staining score was lower (see Fig. 15B).

While the preferred embodiment of the present invention has been described in detail by the examples, it is apparent that modifications and adaptations of the present invention will occur to those skilled in the art. Furthermore, the embodiments of the present invention shall not be interpreted to be restricted by the examples or figures only. It is to be expressly understood, however, that such modifications and adaptations are within the scope of the present invention, as set forth in the following claims. For instance, features illustrated or described as part of one embodiment can be used on another embodiment to yield a still further embodiment. Thus, it is intended that the present invention cover such modifications and variations as come within the scope of the claims and their equivalents.

## Claims

1. An ophthalmic composition including a mixture of lipid nano-dispersion and gel substrate, wherein the lipid nano-dispersion includes a first lipid, a second lipid, and emulsifier; in which the first lipid exists in form of solid lipid as staring material, and the second lipid exists in form of liquid lipid as staring material.

2. The ophthalmic composition according to claim 1, wherein the solid lipid includes yellow Vaseline and wool fat; preferably, wherein the yellow Vaseline and the wool fat are at weight ratio of (4-10) : 1; more preferably, the yellow Vaseline and the wool fat are at weight ratio of 8 : 1.

3. The ophthalmic composition according to claim 1 or 2, wherein, based on the total volume of the ophthalmic composition, the ophthalmic composition includes 0.1% ~ 6% (w/v) of the first lipid; preferably, the ophthalmic composition includes 0.1% ~ 0.5% (w/v) of the first lipid.

4. The ophthalmic composition according to claim 1 or 2, wherein the liquid lipid includes at least one of medium-chain triglycerides, propylene glycol dicaprylate/dicaprate, naphthenic mineral oil, mineral oil, castor oil, polyoxyethylene castor oil, and polyoxyethylene hydrogenated castor oil; preferably, the liquid lipid includes medium-chain triglycerides; more preferably, the medium-chain triglycerides include caprylic/capric triglyceride.

5. The ophthalmic composition according to claim 1 or 2, wherein, based on the total volume of the ophthalmic composition, the ophthalmic composition includes 0.1% ~ 10% (w/v) of the second lipid; preferably, the ophthalmic composition includes 0.25% ~ 8% (w/v) of the second lipid.

6. The ophthalmic composition according to claim 1 or 2, wherein the emulsifier includes at least one oil-phase emulsifier, and optionally, at least one aqueous-phase emulsifier.

7. The ophthalmic composition according to claim 6, wherein
(i) based on the total volume of the ophthalmic composition, the ophthalmic composition includes 0.1% ~ 6% (w/v) of the at least one oil-phase emulsifier; preferably, the ophthalmic composition includes 0.2% ~ 3.5% (w/v) of the at least one oil-phase emulsifier; or
(ii) the at least one oil-phase emulsifier includes at least one of Egg yolk phosphatidylcholine, Soybean phospholipids, TegoCare, Hydrogenated soybean phosphatidylcholine, Myverol, and Span; preferably, the at least one oil-phase emulsifier includes Egg yolk phosphatidylcholine; or
(iii) based on the total volume of the ophthalmic composition, the ophthalmic composition includes 0.1% ~ 8% (w/v) of the at least one aqueous-phase emulsifier; preferably, the ophthalmic composition includes 0.25% ~ 6% (w/v) of the at least one aqueous-phase emulsifier; or
(iv) the at least one aqueous-phase emulsifier includes at least one of Poloxamer 188, Tween, sodium cholate, Sodium deoxycholate, Cremophor EL, and Solutol® HS; preferably, the at least one aqueous-phase emulsifier includes Poloxamer 188.

8. The ophthalmic composition according to claim 1 or 2, wherein the gel substrate includes a gel-forming agent dispersed in a buffer solution; preferably, the gel substrate further includes a water-retaining agent.

9. The ophthalmic composition according to claim 8, wherein
(i) the gel-forming agent includes at least one of polyvinyl pyrrolidone, polyacrylates, and polysaccharides; preferably, the gel-forming agent includes polyvinyl pyrrolidone; or
(ii) based on the total volume of the ophthalmic composition, the ophthalmic composition includes 0.2% ~ 3% (w/v) of the gel-forming agent; preferably, the ophthalmic composition includes 1% ~ 1.8% (w/v) of the gel-forming agent; or
(iii) the water-retaining agent includes at least one of polyethylene glycol, glycerin, hyaluronic acid, chitosan, chondroitin sulfate, and sodium Hyaluronate; preferably, the water-retaining agent includes glycerin; or
(iv) based on the total volume of the ophthalmic composition, the ophthalmic composition includes 0.1 % ~ 5% (w/v) of the water-retaining agent; preferably, the ophthalmic composition includes 0.1% ~ 3% (w/v) of the water-retaining agent; or
(v) the buffer solution includes at least one of borate saline buffer solution, phosphate buffer solution, Gifford's buffer solution, sodium acetate-boric acid buffer solution, Atking and parting buffer solution, and Feldman buffer solution; preferably, the buffer solution includes borate saline buffer solution.

10. The ophthalmic composition according to claim 1 or 2, wherein
(i) the ophthalmic composition has a pH in a range of 4-9; preferably, the ophthalmic composition has a pH in a range of 6~9; more preferably, the ophthalmic composition has a pH in a range of 6~8; or
(ii) the ophthalmic composition has a lipid particle size in a range of 20 - 500 nm; preferably, the ophthalmic composition has a lipid particle size in a range of 50 - 400 nm; more preferably, the ophthalmic composition has a lipid particle size in a range of 50 ~ 200 nm; or
(iii) the ophthalmic composition has a Polydispersity Index less than 0.5; preferably, the ophthalmic composition has a Polydispersity Index less than 0.4; more preferably, the ophthalmic composition has a Polydispersity Index less than 0.3; or
(iv) the ophthalmic composition has a zeta potential in a range of ± 5-50 mV; preferably, the ophthalmic composition has a zeta potential in a range of ± 10-40 mV; more preferably, the ophthalmic composition has a zeta potential in a range of ± 20-30 mV.

11. The ophthalmic composition according to claim 1 or 2, wherein the ophthalmic composition includes at least one drug; preferably, the at least one drug includes at least one of Cyclosporine, Tacrolimus, Flurbiprofen, Triamcinolone Acetonide, Tobramycin, Difluprednate, and Latanoprost.

12. A method for preparing the ophthalmic composition according to any one of claims 1 to 11, wherein the method includes:
I) independently preparing a lipid nano-dispersion and a gel substrate,
wherein the preparation of the lipid nano-dispersion includes:
i) mixing an oil phase dispersion containing a solid lipid, a liquid lipid, an oil-phase emulsifier dispersed in an organic solvent, preferably at a temperature in a range of 60 - 90°C, with an aqueous phase solution containing a aqueous-phase emulsifier dissolved in water, preferably at a temperature in a range of 60 - 90°C, to obtain a preemulsion, preferably for 3-10min;
ii) homogenizing the preemulsion, preferably under a pressure in a range of 500 - 1500 bar for 6-20 cycles;
iii) removing the organic solvent from the homogenized preemulsion by evaporation; and
iv) solidifying the preemulsion, preferably at a temperature in a range of 15 - 30°C for 10-60 min and ice bathed for 5-30 min, to obtain the lipid nano-dispersion; and
wherein the preparation of the gel substrate includes: dispersing a gel-forming agent and a water-retaining agent in a buffer solution; and
II) mixing the lipid nano-dispersion and gel substrate.

13. The ophthalmic composition according to any one of claims 1 to 11 for use in preventing or treating dry eye syndrome, ocular inflammation, cystoid macular edema, or Glaucoma.

## Patentansprüche

1. Ophthalmische Zusammensetzung, die eine Mischung aus Lipid-Nanodispersion und Gelsubstrat umfasst, wobei die Lipid-Nanodispersion ein erstes Lipid, ein zweites Lipid und einen Emulgator umfasst; worin das erste Lipid in Form eines festen Lipids als Ausgangsmaterial vorliegt und das zweite Lipid in Form eines flüssigen Lipids als Ausgangsmaterial vorliegt.

2. Die ophthalmische Zusammensetzung nach Anspruch 1, wobei das feste Lipid gelbe Vaseline und Wollfett umfasst; bevorzugt, wobei die gelbe Vaseline und das Wollfett in einem Gewichtsverhältnis von (4-10) : 1 stehen; stärker bevorzugt, wobei die gelbe Vaseline und das Wollfett in einem Gewichtsverhältnis von 8 : 1 stehen.

3. Die ophthalmische Zusammensetzung nach Anspruich 1 oder 2, wobei, bezogen auf das Gesamtvolumen der ophthalmischen Zusammensetzung, die ophthalmische Zusammensetzung 0,1 - 6 Gew.-% des ersten Lipids umfasst; bevorzugt, wobei die ophthalmische Zusammensetzung 0,1 ~ 0,5 Gew.-% des ersten Lipids umfasst.

4. Die ophthalmische Zusammensetzung nach Anspruch 1 oder 2, wobei das flüssige Lipid mindestens eines von mittelkettigen Triglyceriden, Propylenglykoldicaprylat/- dicaprat, naphthenisches Mineralöl, Mineralöl, Rizinusöl, Polyoxyethylen-Rizinusöl und Polyoxyethylen-hydriertes Rizinusöl umfasst; bevorzugt, wobei das flüssige Lipid mittelkettige Triglyceride umfasst; stärker bevorzugt, wobeidie mittelkettigen Triglyceride Capryl-/Caprintriglyceride umfassen.

5. Die ophthalmische Zusammensetzung nach Anspruch 1 oder 2, wobei, bezogen auf das Gesamtvolumen der ophthalmischen Zusammensetzung, die ophthalmische Zusammensetzung 0,1 - 10 Gew.-% des zweiten Lipids umfasst; bevorzugt, wobei die ophthalmische Zusammensetzung 0,25 - 8 Gew.-% des zweiten Lipids umfasst.

6. Die ophthalmische Zusammensetzung nach Anspruch 1 oder 2, wobei der Emulgator mindestens einen Ölphasenemulgator, und optional, mindestens einen wässerigen Phasenemulgator umfasst.

7. Die ophthalmische Zusammensetzung nach Anspruch 6, wobei:
(i) bezogen auf das Gesamtvolumen der ophthalmischen Zusammensetzung, die ophthalmische Zusammensetzung 0,1 - 6 Gew.-% des mindestens einen Ölphasenemulgators umfasst; bevorzugt, wobei die ophthalmische Zusammensetzung 0,2 ~ 3,5 Gew.-% des mindestens einen Ölphasenemulgators umfasst; oder
(ii) der mindestens eine Ölphasenemulgator mindestens eines von Eigelbphosphatidylcholin, Sojabohnenphospholipide, TegoCare, hydriertes Sojabohnenphosphatidylcholin, Myverol und Span umfasst; bevorzugt, wobei der mindestens eine Ölphasenemulgator Eigelbphosphatidylcholin umfasst; oder
(iii) bezogen auf das Gesamtvolumen der ophthalmischen Zusammensetzung, die ophthalmische Zusammensetzung 0,1 - 8 Gew.-% des mindestens einen wässerigen Phasenemulgators umfasst; bevorzugt, wobei die ophthalmische Zusammensetzung 0,25 - 6 Gew.-% des mindestens einen wässerigen Phasenemulgators umfasst; oder
(iv) der mindestens eine wässerige Ölphasenemulgator mindestens eines von Poloxamer 188, Tween, Natriumcholat, Natriumdesoxycholat, Cremophor EL und Solutol® HS umfasst; bevorzugt, wobei der mindestens eine wässerige Phasenemulgator Poloxamer 188 umfasst.

8. Die ophthalmische Zusammensetzung nach Anspruch 1 oder 2, wobei das Gelsubstrat ein gelbildendes Mittel umfasst, das in einer Pufferlösung dispergiert ist; bevorzugt, wobei das Gelsubstrat ferner ein Wasserrückhaltemittel umfasst.

9. Die ophthalmische Zusammensetzung nach Anspruch 8, wobei:
(i) das gelbildende Mittel mindestens eines von Polyvinylpyrrolidone, Polyacrylate und Polysaccharide umfasst; bevorzugt, wobei das gelbildende Mittel Polyvinylpyrrolidon umfasst; oder
(ii) bezogen auf das Gesamtvolumen der ophthalmischen Zusammensetzung, die ophthalmische Zusammensetzung 0,2 - 3 Gew.-% des gelbildenden Mittels umfasst; bevorzugt, wobei die ophthalmische Zusammensetzung 1 - 1,8 Gew.-% des gelbildenden Mittels umfasst; oder
(iii) das Wasserrückhaltemittel mindestens eines von Polyethylenglycol, Glycerin, Hyaluronsäure, Chitosan, Chondroitinsulfat und Natriumhyaluronat umfasst; bevorzugt, wobei das Wasserrückhaltemittel Glycerin umfasst; oder
(iv) bezogen auf das Gesamtvolumen der ophthalmischen Zusammensetzung, die ophthalmische Zusammensetzung 0,1 - 5 Gew.-% des Wasserrückhaltemittels umfasst; bevorzugt, wobei die ophthalmische Zusammensetzung 0,1 - 3 Gew.-% des Wasserrückhaltemittels umfasst; oder
(v) die Pufferlösung mindestens eines von Boratsalzpufferlösung, Phosphatpufferlösung, Gifford-Pufferlösung, Natriumacetat-Borsäure-Pufferlösung, Atkins-Pantin-Pufferlösung und Feldman-Pufferlösung umfasst; bevorzugt, wobei die Pufferlösung Boratsalzpufferlösung umfasst.

10. Die ophthalmische Zusammensetzung nach Anspruch 1 oder 2, wobei:
(i) die ophthalmische Zusammensetzung einen pH-Wert in einem Bereich von 4 ~ 9 aufweist; bevorzugt, wobei die ophthalmische Zusammensetzung einen pH-Wert im Bereich von 6~9 aufweist; stärker bevorzugt, wobei die ophthalmische Zusammensetzung einen pH-Wert im Bereich von 6 ~ 8 aufweist; oder
(ii) die ophthalmische Zusammensetzung eine Lipidteilchengröße in einem Bereich von 20 - 500 nm aufweist; bevorzugt, wobei die ophthalmische Zusammensetzung eine Lipidteilchengröße in einem Bereich von 50 - 400 nm hat; stärker bevorzugt, wobei die ophthalmische Zusammensetzung eine Lipidteilchengröße in einem Bereich von 50 - 200 nm aufweist; oder
(iii) die ophthalmische Zusammensetzung einen Polydispersitätsindex von weniger als 0,5 aufweist; bevorzugt, wobei die ophthalmische Zusammensetzung einen Polydispersitätsindex von weniger als 0,4 aufweist; stärker bevorzugt, wobei die ophthalmische Zusammensetzung einen Polydispersitätsindex von weniger als 0,3 aufweist; oder
(iv) die ophthalmische Zusammensetzung ein Zetapotential in einem Bereich von ± 5 - 50 mV aufweist; bevorzugt, wobei die ophthalmische Zusammensetzung ein Zetapotential in einem Bereich von ± 10 - 40 mV aufweist; stärker bevorzugt, wobei die ophthalmische Zusammensetzung ein Zetapotential in einem Bereich von ± 20 - 30 mV aufweist.

11. Die ophthalmische Zusammensetzung nach Anspruch 1 oder 2, wobei die ophthalmische Zusammensetzung mindestens einen Wirkstoffumfasst; bevorzugt, wobei der mindestens eine Wirkstoff mindestens einer von Cyclosporin, Tacrolimus, Flurbiprofen, Triamcinolonacetonid, Tobramycin, Difluprednat und Latanoprost umfasst.

12. Verfahren zur Herstellung der ophthalmischen Zusammensetzung nach einem der Ansprüche 1 bis 11, wobei das Verfahren umfasst:
I) unabhängiges Herstellen einer Lipid-Nanodispersion und eines Gelsubstrats, wobei die Herstellung der Lipid-Nanodispersion umfasst:
i) Mischen eine Ölphasendispersion, die ein festes Lipid, ein flüssiges Lipid und einen Ölphasenemulgator, dispergiert in einem organischen Lösungsmittel, enthält, bevorzugt bei einer Temperatur im Bereich von 60 ~ 90 °C, mit einer wässrigen Phasenlösung, die einen wässrigen Phasenemulgator gelöst in Wasser enthält, bevorzugt bei einer Temperatur im Bereich von 60 ~ 90 °C, um eine Voremulsion zu erhalten, bevorzugt für 3 ~ 10 Minuten;
ii) Homogenisieren der Voremulsion, bevorzugt unter einem Druck in einem Bereich von 500 ~ 1500 bar für 6 ~ 20 Zyklen;
iii) Entfernen des organischen Lösungsmittels aus der homogenisierten Voremulsion durch Verdampfen; und
iv) Verfestigen der Voremulsion, bevorzugt bei einer Temperatur im Bereich von 15 -30 °C für 10 - 60 Minuten und im Eisbad für 5 ~ 30 Minuten, um die Lipid-Nanodispersion zu erhalten; und wobei die Herstellung des Gelsubstrats umfasst: Dispergieren eines gelbildenden Mittels und eines wasserrückhaltenden Mittels in einer Pufferlösung; und
II) Mischen der Lipid-Nanodispersion und des Gelsubstrats.

13. Die ophthalmische Zusammensetzung nach einem der Ansprüche 1 bis 11 zur Verwendung in der Vorbeugung oder Behandlung eines Syndroms des trockenen Auges, einer Augenentzündung, eines zystoiden Makulaödems oder eines Glaukoms.

## Revendications

1. Composition ophtalmique comprenant un mélange de nano-dispersion de lipides et de substrat de gel, la nano-dispersion de lipides comprenant un premier lipide, un second lipide et un émulsifiant ; dans ladite composition, le premier lipide est présent sous la forme d'un lipide solide comme matière première, et le second lipide est présent sous la forme d'un lipide liquide comme matière première.

2. Composition ophtalmique selon la revendication 1, dans laquelle le lipide solide comprend de la vaseline jaune et de la lanoline, de préférence la vaseline jaune et la lanoline étant présentes dans un rapport pondéral de (4-10):1, plus préférablement la vaseline jaune et la lanoline étant présentes dans un rapport pondéral de 8:1.

3. Composition ophtalmique selon la revendication 1 ou 2, dans laquelle, sur la base du volume total de la composition ophtalmique, la composition ophtalmique comprend 0,1 % ~6 % (p/v) du premier lipide ; de préférence, la composition ophtalmique comprend 0,1 % ~ 0,5 % (p/v) du premier lipide.

4. Composition ophtalmique selon la revendication 1 ou 2, dans laquelle le lipide liquide comprend au moins un composé parmi les triglycérides à chaîne moyenne, le dicaprylate/dicaprate de propylène glycol, l'huile minérale naphténique, l'huile minérale, l'huile de ricin, l'huile de ricin polyoxyéthylénée, l'huile de ricin hydrogénée polyoxyéthylénée ; de préférence, le lipide liquide comprend des triglycérides à chaîne moyenne ; plus préférablement, les triglycérides à chaîne moyenne comprennent un triglycéride caprylique/caprique.

5. Composition ophtalmique selon la revendication 1 ou 2, dans laquelle, sur la base du volume total de la composition ophtalmique, la composition ophtalmique comprend 0,1 % ~ 10 % (p/v) du second lipide ; de préférence, la composition ophtalmique comprend 0,25 % ~ 8 % (p/v) du second lipide.

6. Composition ophtalmique selon la revendication 1 ou 2, dans laquelle l'émulsifiant comprend au moins un émulsifiant en phase huileuse et, facultativement, au moins un émulsifiant en phase aqueuse.

7. Composition ophtalmique selon la revendication 6, dans laquelle
(i) sur la base du volume total de la composition ophtalmique, la composition ophtalmique comprend 0,1 % ~ 6 % (p/v) de l'au moins un émulsifiant en phase huileuse ; de préférence, la composition ophtalmique comprend 0,2 % ~ 3,5 % (p/v) de l'au moins un émulsifiant en phase huileuse ; ou
(ii) l'au moins un émulsifiant en phase huileuse comprend au moins un composé parmi la phosphatidylcholine de jaune d'oeuf, les phospholipides de soja, le TegoCare, la phosphatidylcholine de soja hydrogénée, le Myverol et le Span ; de préférence, l'au moins un émulsifiant en phase huileuse comprend de la phosphatidylcholine de jaune d'oeuf ; ou
(iii) sur la base du volume total de la composition ophtalmique, la composition ophtalmique comprend 0,1 % ~ 8 % (p/v) de l'au moins un émulsifiant en phase aqueuse ; de préférence, la composition ophtalmique comprend 0,25 % ~ 6 % (p/v) de l'au moins un émulsifiant en phase aqueuse ; ou
(iv) l'au moins un émulsifiant en phase aqueuse comprend au moins un composé parmi le Poloxamer 188, Tween, le cholate de sodium, le désoxycholate de sodium, le Cremophor EL, et le Solutol® HS ;
de préférence l'au moins un émulsifiant en phase aqueuse comprend le Poloxamer 188.

8. Composition ophtalmique selon la revendication 1 ou 2, dans laquelle le substrat de gel comprend un agent gélifiant dispersé dans une solution tampon ; de préférence, le substrat de gel comprend en outre un agent de rétention d'eau.

9. Composition ophtalmique selon la revendication 8, dans laquelle
(i) l'agent gélifiant comprend au moins un composé parmi la polyvinylpyrrolidone, les polyacrylates et les polysaccharides ; de préférence, l'agent gélifiant comprend de la polyvinylpyrrolidone ; ou
(ii) sur la base du volume total de la composition ophtalmique, la composition ophtalmique comprend 0,2 % ~ 3 % (p/v) de l'agent gélifiant ; de préférence, la composition ophtalmique comprend 1 % ~ 1,8 % (p/v) de l'agent gélifiant ; ou
(iii) l'agent de rétention d'eau comprend au moins un composé parmi le polyéthylène glycol, le glycérol, l'acide hyaluronique, le chitosane, le sulfate de chondroïtine et le hyaluronate de sodium ; de préférence, l'agent de rétention d'eau comprend du glycérol ; ou
(iv) sur la base du volume total de la composition ophtalmique, la composition ophtalmique comprend 0,1 % ~ 5 % (p/v) de l'agent de rétention d'eau ; de préférence, la composition ophtalmique comprend 0,1 % ~ 3 % (p/v) de l'agent de rétention d'eau ; ou
(v) la solution tampon comprend au moins une solution parmi une solution tampon de borate salin, une solution tampon de phosphate, une solution tampon de Gifford, une solution tampon d'acétate de sodium-acide borique, une solution tampon d'Atkins et de Pantin et une solution tampon de Feldman ; de préférence, la solution tampon comprend une solution tampon de borate salin.

10. Composition ophtalmique selon la revendication 1 ou 2, dans laquelle
(i) la composition ophtalmique a un pH compris entre 4 et 9 ; de préférence, la composition ophtalmique a un pH compris entre 6 et 9 ; plus préférablement, la composition ophtalmique a un pH compris entre 6 et 8 ; ou
(ii) la composition ophtalmique a une taille de particule lipidique comprise entre 20 et 500 nm ; de préférence, la composition ophtalmique a une taille de particule lipidique comprise entre 50 et 400 nm ; plus préférablement, la composition ophtalmique a une taille de particule lipidique comprise entre 50 et 200 nm ;
ou
(iii) la composition ophtalmique a un indice de polydispersité inférieur à 0,5 ; de préférence, la composition ophtalmique a un indice de polydispersité inférieur à 0,4 ; plus préférablement, la composition ophtalmique a un indice de polydispersité inférieur à 0,3; ou
(iv) la composition ophtalmique a un potentiel zêta compris entre ± 5 et 50 mV ; de préférence, la composition ophtalmique a un potentiel zêta compris entre ± 10 et 40 mV ; plus préférablement, la composition ophtalmique a un potentiel zêta compris entre ± 20 et 30 mV.

11. Composition ophtalmique selon la revendication 1 ou 2, dans laquelle la composition ophtalmique comprend au moins un médicament ; de préférence, l'au moins un médicament comprend au moins un composé parmi la cyclosporine, le tacrolimus, le flurbiprofène, l'acétonide de triamcinolone, la tobramycine, le difluprednate et le latanoprost.

12. Procédé de préparation de la composition ophtalmique selon l'une quelconque des revendications 1 à 11, dans lequel le procédé comprend :
I) la préparation de manière indépendante d'une nano-dispersion de lipides et d'un substrat de gel,
la préparation de la nano-dispersion de lipides comprenant :
i) le mélange d'une dispersion en phase huileuse contenant un lipide solide, un lipide liquide, un émulsifiant en phase huileuse dispersé dans un solvant organique, de préférence à une température comprise entre 60 et 90 °C, avec une solution en phase aqueuse contenant un émulsifiant en phase aqueuse dissous dans de l'eau, de préférence à une température comprise entre 60 et 90 °C, pour obtenir une pré-émulsion, de préférence pendant 3 ~ 10 minutes ;
ii) l'homogénéisation de la pré-émulsion, de préférence à une pression comprise entre 500 et 1500 bars pendant 6 ~ 20 cycles ;
iii) l'élimination du solvant organique de la pré-émulsion homogénéisée par évaporation ; et
iv) la solidification de la pré-émulsion, de préférence à une température comprise entre 15 et 30 °C pendant 10 - 60 minutes et baignée dans de la glace pendant 5 ~ 30 minutes, pour obtenir la nano-dispersion de lipides ; et
la préparation du substrat de gel comprenant : la dispersion d'un agent gélifiant et d'un agent de rétention d'eau dans une solution tampon ; et
II) le mélange de la nano-dispersion de lipides et le substrat de gel.

13. Composition ophtalmique selon l'une quelconque des revendications 1 à 11, destinée à prévenir ou traiter le syndrome de l'oeil sec, l'inflammation oculaire, l'oedème maculaire cystoïde ou le glaucome.
